# EUROPEAN PATENT SPECIFICATION

(11) **EP 1 083 873 B1**
(45) Date of publication and mention of the grant of the patent: **09.08.2006**
(21) Application number: 98925157.4
(22) Date of filing: 03.06.1998
(51) Int. Cl.: A61K 8/00, A61K 31/40, A61K 31/44, A61K 31/445, A61K 31/425, A61K 31/415, A61K 31/535, A61K 31/54

(54) **HETEROCYCLIC THIOESTER AND KETONE HAIR GROWTH COMPOSITIONS AND USES**
HAARWUCHSZUSAMMENSETZUNGEN BASIEREND AUF EINEM HETEROZYKLISCHEN ESTER UND KETON UND ANWENDUNGEN
COMPOSITIONS POUR LA CROISSANCE DES CHEVEUX A BASE DE KETONES ET DE THIOESTERS HETEROCYCLIQUES ET UTILISATION DE CES COMPOSITIONS

(43) Date of publication of application: 21.03.2001
(73) Proprietor: MGI GP, Inc., Baltimore, MD 21224 (US)
(72) Inventor: HAMILTON, Gregory, S., Catonsville, MD 21228 (US); STEINER, Joseph, P., Mt. Airy, Maryland, 21771 (US)
(74) Representative: Bausch, Thorsten
(86) International application number: PCT/US1998/011251
(87) International publication number: WO 1999/062488

(56) References cited:
- EP-A- 0 423 714
- EP-A- 0 471 135
- WO-A-93/14762
- WO-A-98/13343

## Description

### 1. Field of Invention

This invention relates to the use of small molecule heterocyclic thioesters and ketones for preparing medicaments for treating alopecia or promoting hair growth.

### 2. Description of Related Art

Hair loss occurs in a variety of situations. These situations include male pattern alopecia, alopecia senilis, alopecia areata, diseases accompanied by basic skin lesions or tumors, and systematic disorders such as nutritional disorders and internal secretion disorders. The mechanisms causing hair loss are very complicated, but in some instances can be attributed to aging, genetic disposition, the activation of male hormones, the loss of blood supply to hair follicles, and scalp abnormalities.

The immunosuppressant drugs FK506, rapamycin and cyclosporin are well known as potent T-cell specific immunosuppressants, and are effective against graft rejection after organ transplantation. It has been reported that topical, but not oral, application of FK506 (Yamamoto et al., J. Invest. Dermatol., 1994, 102, 160-164; Jiang et al., J. Invest. Dermatol. 1995, 104, 523-525) and cyclosporin (Iwabuchi et al., J. Dermatol. Sci. 1995, 9, 64-69) stimulates hair growth in a dose-dependent manner. One form of hair loss, alopecia areata, is known to be associated with autoimmune activities; hence, topically administered immunomodulatory compounds are expected to demonstrate efficacy for treating that type of hair loss. The hair growth stimulating effects of FK506 have been the subject of an international patent filing covering FK506 and structures related thereto for hair growth stimulation (Honbo et al., EP 0 423 714 A2). Honbo et al. discloses the use of relatively large tricyclic compounds, known for their immunosuppressive effects, as hair revitalizing agents.

The hair growth and revitalization effects of FK506 and related agents are disclosed in many U.S. patents (Goulet et al., U.S. Patent No. 5,258,389; Luly et al., U.S. Patent No. 5,457,111; Goulet et al., U.S. Patent No. 5,532,248; Goulet et al., U.S. Patent No. 5,189,042; and Ok et al., U.S. Patent No. 5,208,241; Rupprecht et al., U.S. Patent No. 5,284,840; Organ et al., U.S. Patent No. 5,284,877). These patents claim FK506 related compounds. Although they do not claim methods of hair revitalization, they disclose the known use of FK506 for effecting hair growth. Similar to FK506 (and the claimed variations in the Honbo et al. patent), the compounds claimed in these patents are relatively large. Further, the cited patents relate to immunomodulatory compounds for use in autoimmune related diseases, for which FK506's efficacy is well known.

Other U.S. patents disclose the use of cyclosporin and related compounds for hair revitalization (Hauer et al., U.S. Patent No. 5,342,625; Eberle, U.S. Patent No. 5,284,826; Hewitt et al., U.S. Patent No. 4,996,193). These patents also relate to compounds useful for treating autoimmune diseases and cite the known use of cyclosporin and related immunosuppressive compounds for hair growth.

However, immunosuppressive compounds by definition suppress the immune system and also exhibit other toxic side effects. Accordingly, there is a need for non-immunosuppressant, small molecule compounds which are useful as hair revitalizing compounds.

Hamilton and Steiner disclose in U.S. Patent No. 5,614,547 novel pyrrolidine carboxylate compounds which bind to the immunophilin FKBP12 and stimulate nerve growth, but which lack immunosuppressive effects. Unexpectedly, it has been discovered that these non-immunosuppressant compounds promote hair growth with an efficacy similar to FK506. Yet their novel small molecule structure and non-immunosuppressive properties differentiate them from FK506 and related immunosuppressive compounds found in the prior art.

WO-A-93/14762 relates to a pharmaceutical composition comprising a carrier and a heterocyclic compound comprising a ketone molecule portion. This document further discloses a method for preparing such compounds starting with a steroid ester which is converted to a thio-pyrrodyl ester as an intermediate product.

### SUMMARY OF THE INVENTION

The present invention relates to the use of heterocyclic thioesters or ketones for the preparation of pharmaceutical compositions or medicaments for treating alopecia or promoting hair growth.

Specifically, the present invention provides the use of a compound for the preparation of a pharmaceutical composition or medicament for treating alopecia or promoting hair growth in an animal in need thereof, wherein said compound is of formula V 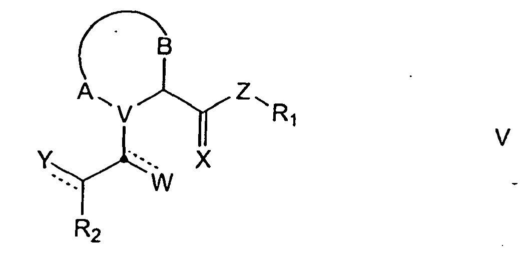 or a pharmaceutically acceptable salt, ester, or solvate thereof, wherein:
V is C, N, or S;
A and B, taken together with V and the carbon atom to which they are respectively attached, form a 5-7 membered saturated or unsaturated heterocyclic ring containing one or more heteroatom(s) independently selected from the group consisting of O, S, SO, SO₂, N, NH, and NR₄;
R₄ is either C₁-C₉ straight or branched chain alkyl, C₂-C₉ straight or branched chain alkenyl, C₃-C₉ cycloalkyl, C₅-C₇ cycloalkenyl, or Ar₃,
   wherein R₄ is either unsubstituted or substituted with one or more substituent(s) independently selected from the group consisting of halo, haloalkyl, carbonyl, carboxy, hydroxy, nitro, trifluoromethyl, C₁₋C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄;
Ar₃ and Ar₄ are independently an alicyclic or aromatic, mono-, bi- or tricyclic, carbo- or heterocyclic ring,
   wherein the individual ring size is 5-8 members,
   and wherein said heterocyclic ring contains 1-6 heteroatom(s) independently selected from the group consisting of O, N, and S;
X is either O or S;
Z is either S, CH₂, CHR₁ or CR₁R₃;
W and Y are independently O, S, CH₂ or H₂;
R, and R₃ are independently C₁-C₆ straight or branched chain alkyl or C₂-C₆ straight or branched chain alkenyl,
   wherein said alkyl or alkenyl is substituted with one or more substituent(s) independently selected from the group consisting of (Ar₁) ₙ, C₁-C₆ straight or branched chain alkyl or C₂₋C₆ straight or branched chain alkenyl substituted with (Ar₁)ₙ, C₃-C₈ cycloalkyl, C₁₋C₆ straight or branched chain alkyl or C₂-C₆ straight or branched chain alkenyl substituted with C₃-C₈ cycloalkyl, and Ar₂; n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁,
   wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring,
wherein said ring is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino,
wherein the individual ring size is 5-8 members,
and wherein the heterocyclic ring has 1-6 heteroatom(s) independently selected from the group consisting of O, N, and S.

The heterocyclic thioesters and ketones have an affinity for FKBP-type immunophilins, particularly FKBP12, and do not exert any significant immunosuppressive activity.

### BRIEF DESCRIPTION OF THE DRAWINGS

FIG. 1 is a photograph of C57 Black 6 mice before being shaved for the hair regeneration experiment.
FIG. 2 is a photograph of mice treated with a vehicle after six weeks. FIG. 2 shows that less than 3% of the shaved area is covered with new hair growth when the vehicle (control) is administered.
FIG. 3 is a photograph of mice treated with 10 µM of GPI 1046, a related non-immunosuppressive neuroimmunophilin FKBP ligand, after six weeks. FIG. 3 shows the remarkable effects of non-immunosuppressive neuroimmunophilin FKBP ligands, wherein 90% of the shaved area is covered with new hair growth.
FIG. 4 is a photograph of mice treated with 30 µM of GPI 1046 after six weeks. FIG. 4 shows the remarkable ability of non-immunosuppressive neuroimmunophilin FKBP ligands to achieve, essentially, complete hair regrowth in the shaved area.
FIG. 5 is a bar graph depicting the relative hair growth indices for C57 Black 6 mice treated with a vehicle, FK506, and various non-immunosuppressive neuroimmunophilin FKBP ligands, including GPI 1389, 1511, and 1234, 14 days after treatment with each identified compound. Figure 5 demonstrates the remarkable early hair growth promoted by a wide variety of non-immunosuppressive neuroimmunophilin FKBP ligands.

### DETAILED DESCRIPTION OF THE INVENTION

### Definitions

"Alopecia" refers to deficient hair growth and partial or complete loss of hair, including without limitation androgenic alopecia (male pattern baldness), toxic alopecia, alopecia senilis, alopecia areata, alopecia pelada and trichotillomania. Alopecia results when the pilar cycle is disturbed. The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae, and the hairs fall out. Alopecia has a number of etiologies, including genetic factors, aging, local and systemic diseases, febrile conditions, mental stresses, hormonal problems, and secondary effects of drugs.

"GPI 1605" refers to a compound of formula

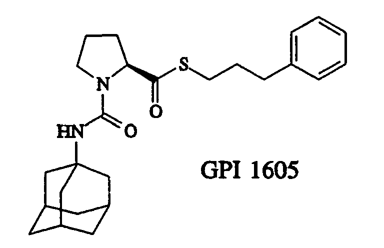

"GPI 1046" refers to 3-(3-pyridyl)-1-propyl (2s)-1-(3,3-dimethyl-1,2-dioxopentyl)-2-pyrrolidinecarboxylate, a compound of formula

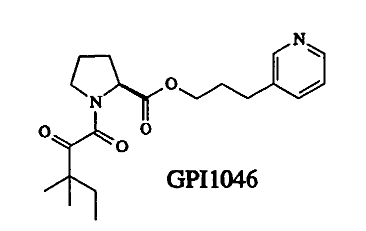

"GPI 1312" refers to a compound of formula

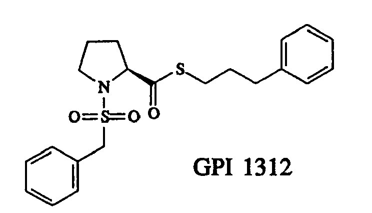

"GPI 1572" refers to a compound of formula

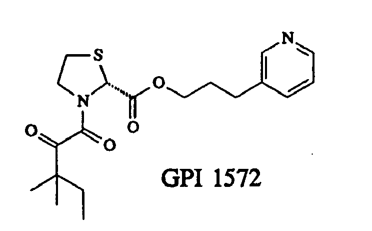

"GPI 1389" refers to a compound of formula

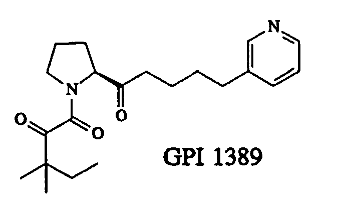

"GPI 1511" refers to a compound of formula

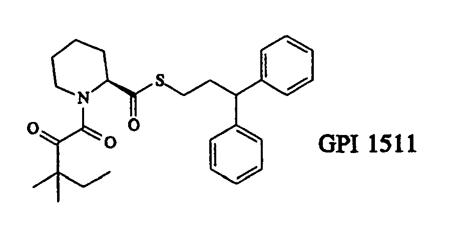

"GPI 1234" refers to a compound of formula

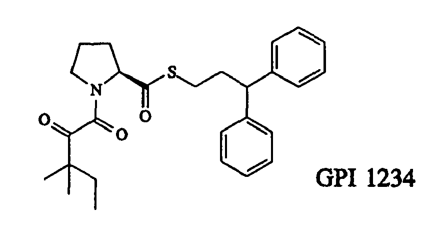

"Isomers" refer to different compounds that have the same molecular formula. "Stereoisomers" are isomers that differ only in the way the atoms are arranged in space. "Enantiomers" are a pair of stereoisomers that are non-superimposable mirror images of each other. "Diastereoisomers" are stereoisomers which are not mirror images of each other. "Racemic mixture" means a mixture containing equal parts of individual enantiomers. "Non-racemic mixture" is a mixture containing unequal parts of individual enantiomers or stereoisomers.

"Pharmaceutically acceptable salt, ester, or solvate" refers to a salt, ester, or solvate of a subject compound which possesses the desired pharmacological activity and which is neither biologically nor otherwise undesirable. A salt, ester, or solvate can be formed with inorganic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride, hydrobromide, hydroiodide, 2-hydroxyethanesulfonate, lactate, maleate, methanesulfonate,naphthylate,2-naphthalenesulfonate, nicotinate, oxalate, sulfate, thiocyanate, tosylate and undecanoate. Examples of base salts, esters, or solvates include ammonium salts; alkali metal salts, such as sodium and potassium salts; alkaline earth metal salts, such as calcium and magnesium salts; salts with organic bases, such as dicyclohexylamine salts; N-methyl-D-glucamine; and salts with amino acids, such as arginine, lysine, and so forth. Also, the basic nitrogen-containing groups can be quarternized with such agents as lower alkyl halides, such as methyl, ethyl, propyl, and butyl chlorides, bromides, and iodides; dialkyl sulfates, such as dimethyl, diethyl, dibutyl, and diamyl sulfates; long chain halides, such as decyl, lauryl, myristyl, and stearyl chlorides, bromides, and iodides; aralkyl halides, such as benzyl and phenethyl bromides; and others. Water or oil-soluble or dispersible products are thereby obtained.

"Pilar cycle" refers to the life cycle of hair follicles, and includes three phases:
(1) the anagen phase, the period of active hair growth which, insofar as scalp hair is concerned, lasts about three to five years;
(2) the catagen phase, the period when growth stops and the follicle atrophies which, insofar as scalp hair is concerned, lasts about one to two weeks; and
(3) the telogen phase, the rest period when hair progressively separates and finally falls out which, insofar as scalp hair is concerned, lasts about three to four months.
Normally 80 to 90 percent of the follicles are in the anagen phase, less than 1 percent being in the catagen phase, and the rest being in the telogen phase. In the telogen phase, hair is uniform in diameter with a slightly bulbous, non-pigmented root. By contrast, in the anagen phase, hair has a large colored bulb at its root.

"Promoting hair growth" refers to maintaining, inducing, stimulating, accelerating, or revitalizing the germination of hair.

"Treating alopecia" refers to:
(i) preventing alopecia in an animal which may be predisposed to alopecia; and/or
(ii) inhibiting, retarding or reducing alopecia; and/or
(iii) promoting hair growth; and/or
(iv) prolonging the anagen phase of the hair cycle; and/or
(v) converting vellus hair to growth as terminal hair. Terminal hair is coarse, pigmented, long hair in which the bulb of the hair follicle is seated deep in the dermis. Vellus hair, on the other hand, is fine, thin, non-pigmented short hair in which the hair bulb is located superficially in the dermis. As alopecia progresses, the hairs change from the terminal to the vellus type.

### Products derived from the Present Invention

The products derived from the use of the present invention can be used to treat alopecia or promote hair growth in an animal, by administering to said animal an effective amount of a heterocyclic thioester or ketone.

The products derived from the present invention are particularly useful for treating male pattern alopecia, alopecia senilis, alopecia areata, alopecia resulting from skin lesions or tumors, alopecia resulting from cancer therapy such as chemotherapy and radiation, and alopecia resulting from systematic disorders such as nutritional disorders and internal secretion disorders.

### HETEROCYCLIC THIOESTERS AND KETONES

The heterocyclic thioesters and ketones used in the present invention are low molecular weight, small molecule compounds having an affinity for FKBF-type immunophilins, such as FKBP12. When a heterocyclic thioester or ketone binds to an FKBP-type immunophilin, it has been found to inhibit the prolyl-peptidyl *cis*-*trans* isomerase, or rotamase, activity of the binding protein. Unexpectedly, the compounds have also been found to stimulate hair growth. These compounds are devoid of any significant immunosuppressive activity. Examples of useful compounds are set forth below.

### FORMULA I

The heterocyclic thioester or ketone may be a compound of formula I 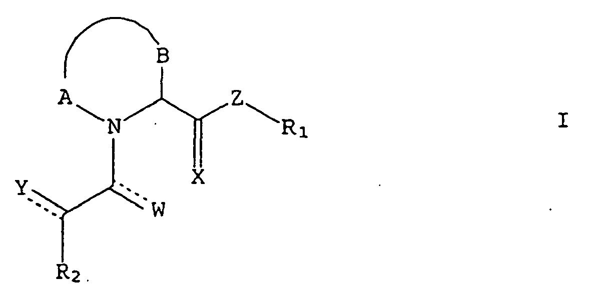 or a pharmaceutically acceptable salt, ester, or solvate thereof, wherein:
A and B, taken together with the nitrogen and carbon atoms to which they are respectively attached, form a 5-7 membered saturated or unsaturated heterocyclic ring containing, in addition to the nitrogen atom, one or more additional O, S, SO, SO₂, N, NH, or NR₂ heteroatom (s) ;
X is either O or S;
Z is either S, CH₂, CHR₁ or CR₁R₃;
W and Y are independently O, S, CH₂ or H₂;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)_{n'} C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl, or Ar₁, wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group including, but no limited to, halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula I, the heterocyclic thioester or ketone is selected from the group consisting of GPI 1389, of the formula 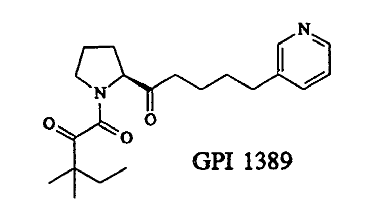 GPI 1511, of the formula 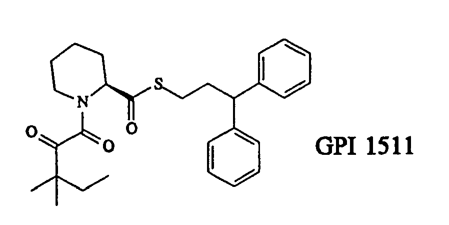 and GPI 1234, of the formula

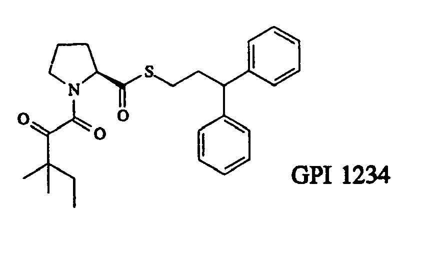

### FORMULA II

The heterocyclic thioester or ketone may also be a compound of formula II 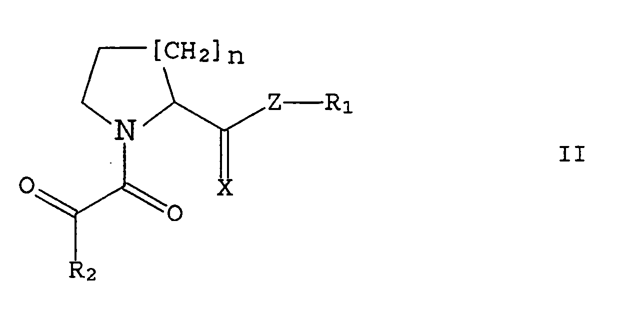 or a pharmaceutically acceptable salt, ester, or solvate thereof, wherein:
n is 1 or 2;
X is O or S;
Z is selected from the group consisting of S, CH₂, CHR₁, and CR₁R₃ ;
R₁ and R₃ are independently selected from the group consisting of C₁-C₅ straight or branched chain alkyl, C₂-C₅ straight or branched chain alkenyl, and Ar₁, wherein said alkyl, alkenyl or Ar₁ is unsubstituted or substituted with one or more substituent(s) independently selected from the group consisting of halo, nitro, C₁-C₆ straight or branched chain alkyl, C₂-C₆ straight or branched chain alkenyl, hydroxy, C₁-C₄ alkoxy, C₂-C₄ alkenyloxy, phenoxy, benzyloxy, amino, and Ar₁;
R₂ is selected from the group consisting of C₁-C₉ straight or branched chain alkyl, C₂-C₉ straight or branched chain alkenyl, C₃-C₈ cycloalkyl, C₅-C₇ cycloalkenyl, and Ar₁; and
Ar₁ is phenyl, benzyl, pyridyl, fluorenyl, thioindolyl or naphthyl, wherein said Ar₁ is unsubstituted or substituted with one or more substituent(s) independently selected from the group consisting of halo, trifluoromethyl, hydroxy, nitro, C₁-C₆ straight or branched chain alkyl, C₂-C₆ straight or branched chain alkenyl, C₁-C₄ alkoxy, C₂-C₄ alkenyloxy, phenoxy, benzyloxy, and amino.

Preferred compounds of formula II are presented in TABLE I.

**TABLE I**

| No. | n | X | Z | R₁ | R₂ |
|---|---|---|---|---|---|
| 1 | 1 | O | CH₂ | 3-Phenylpropyl | 1,1-Dimethylpropyl |
| 2 | 1 | O | CH₂ | 3-(3-Pyridyl)propyl | 1,1-Dimethylpropyl |
| 3 | 1 | O | CH₂ | 3-Phenylpropyl | *tert-*Butyl |
| 4 | 1 | O | CH₂ | 3-(3-Pyridyl)propyl | *tert*-Butyl |
| 5 | 1 | O | CH₂ | 3-(3-Pyridyl)propyl | Cyclohexyl |
| 6 | 1 | O | CH₂ | 3-(3-Pyridyl)propyl | Cyclopentyl |
| 7 | 1 | O | CH₂ | 3-(3-Pyridyl)propyl | Cycloheptyl |
| 8 | 1 | O | CH₂ | 2-(9-Fluorenyl)ethyl | 1,1-Dimethylpropyl |
| 9 | 1 | O | S | 2-Phenethyl | 1,1-Dimethylpropyl |
| 10 | 2 | O | S | 2-Phenethyl | 1,1-Dimethylpropyl |
| 11 | 1 | O | S | Methyl(2-thioindole) | 1,1-Dimethylpropyl |
| 12 | 1 | O | S | 2-Phenethyl | Cyclohexyl |
| 13 | 2 | O | S | 2-Phenethyl | *tert*-Butyl |
| 14 | 2 | O | S | 2-Phenethyl | Phenyl |
| 15 | 1 | O | CH₂ | 3-(4-Methoxyphenyl)propyl | 1,1-Dimethylpropyl |
| 16 | 2 | O | CH₂ | 4-(4-Methoxyphenyl)butyl | 1,1-Dimethylpropyl |
| 17 | 2 | O | CH₂ | 4-Phenylbutyl | 1,1-Dimethylpropyl |
| 18 | 2 | O | CH₂ | 4-Phenylbutyl | Phenyl |
| 19 | 2 | O | CH₂ | 4-Phenylbutyl | Cyclohexyl |
| 20 | 1 | S | CH₂ | 3-Phenylpropyl | 1,1-Dimethylpropyl |
| 21 | 1 | S | S | 2-Phenethyl | 1,1-Dimethylpropyl |
| 22 | 2 | S | CH₂ | 3-Phenylpropyl | 1,1-Dimethylpropyl |
| 23 | 2 | S | S | 2-Phenethyl | 1,1-Dimethylpropyl |
| 24 | 2 | O | CHR₁ | 3-Phenylpropyl | 1,1-Dimethylpropyl |
| 25 | 2 | O | CHR₁ | 3-Phenylpropyl | Cyclohexyl |
| 26 | 2 | O | CHR₁ | 3-Phenylpropyl | Phenyl |
| 27 | 2 | O | CHR₁ | 3-Phenylpropyl | 3,4,5-Trimethoxyphenyl |
| 28 | 1 | O | S | 2-Phenethyl | Cyclopentyl |
| 29 | 2 | O | S | 3-Phenylpropyl | *tert*-Butyl |
| 30 | 1 | O | S | 3-Phenylpropyl | 1,1-Dimethylpropyl |
| 31 | 1 | O | S | 3-(3-Pyridyl)propyl | 1,1-Dimethylpropyl |
| 32 | 1 | O | S | 3-Phenylpropyl | Cyclohexyl |
| 33 | 1 | O | S | 4-Phenylbutyl | Cyclohexyl |
| 34 | 1 | O | S | 4-Phenylbutyl | 1,1-Dimethylpropyl |
| 35 | 1 | O | S | 3-(3-Pyridyl)propyl | Cyclohexyl |
| 36 | 1 | O | S | 3,3-Diphenylpropyl | 1,1-Dimethylpropyl |
| 37 | 1 | O | S | 3,3-Diphenylpropyl | Cyclohexyl |
| 38 | 1 | O | S | 3-(4-Methoxyphenyl) propyl | 1,1-Dimethylpropyl |
| 39 | 2 | O | S | 4-Phenylbutyl | *tert*-Butyl |
| 40 | 2 | O | S | 1,5-Diphenylpentyl | 1,1-Dimethylpropyl |
| 41 | 2 | O | S | 1,5-Diphenylpentyl | Phenyl I |
| 42 | 2 | O | S | 3-(4-Methoxyphenyl) propyl | 1,1-Dimethylpropyl |
| 43 | 2 | O | S | 3-(4-Methoxyphenyl) propyl | Phenyl 1 |
| 44 | 2 | O | S | 3-(1-Naphthyl)propyl | 1,1-Dimethylpropyl |
| 45 | 1 | O | S | 3,3-Di(4-fluoro)phenylpropyl | 1,1-Dimethylpropyl |
| 46 | 1 | O | S | 4,4-Di(4-fluoro)phenylbutyl | 1,1-Dimethylpropyl |
| 47 | 1 | O | S | 3-(1-Naphthyl)propyl | 1,1-Dimethylpropyl |
| 48 | 1 | O | S | 2,2-Diphenylethyl | 1,1-Dimethylpropyl |
| 49 | 2 | O | S | 2,2-Diphenylethyl | 1,1-Dimethylpropyl |
| 50 | 2 | O | S | 3,3-Diphenylpropyl | 1,1-Dimethylpropyl |
| 51 | 1 | O | S | 3-(4-{Trifluoromethyl}-phenyl)propyl | 1,1-Dimethylpropyl |
| 52 | 1 | O | S | 3-(2-Naphthyl)propyl | 1,1-Dimethylpropyl |
| 53 | 2 | O | S | 3-(1-Naphthyl)propyl | 1,1-Dimethylpropyl |
| 54 | 1 | O | S | 3-(3-Chloro)phenylpropyl | 1,1-Dimethylpropyl |
| 55 | 1 | O | S | 3-(3-{Trifluoromethyl}-phenyl)propyl | 1,1-Dimethylpropyl |
| 56 | 1 | O | S | 3-(2-Biphenyl)propyl | 1,1-Dimethylpropyl |
| 57 | 1 | O | S | 3-(2-Fluorophenyl)propyl | 1,1-Dimethylpropyl |
| 58 | 1 | O | S | 3-(3-Fluorophenyl)propyl | 1,1-Dimethylpropyl |
| 59 | 2 | O | S | 4-Phenylbutyl | 1,1-Dimethylpropyl |
| 60 | 2 | O | S | 3-Phenylpropyl | 1,1-Dimethylpropyl |
| 61 | 1 | O | S | 3-(2-Chloro)phenylpropyl | 1,1-Dimethylpropyl |
| 62 | 2 | O | S | 3-(3-Chloro)phenylpropyl | 1,1-Dimethylpropyl |
| 63 | 2 | O | S | 3-(2-Fluoro)phenylpropyl | 1,1-Dimethylpropyl |
| 64 | 2 | O | S | 3-(3-Fluoro)phenylpropyl | 1,1-Dimethylpropyl |
| 65 | 1 | O | S | 3-(2,5-Dimethoxyphenyl)-propyl | 1,1-Dimethylpropyl |
| 66 | 1 | O | CH₂ | 3-Phenylpropyl | Cyclohexyl |
| 67 | 1 | O | CH₂ | 3-Phenylethyl | *tert-*Butyl |
| 68 | 2 | O | CH₂ | 4-Phenylbutyl | Cyclohexyl |
| 69 | 2 | O | CHR₁ | 2-Phenylethyl 1 | *tert-*Butyl |
| 70 | 1 | O | CH₂ | 3,3-Di(4-fluorophenyl)-propyl 1 | 1,1-Dimethylpropyl |
| 71 | 2 | O | CH₂ | 3-Phenylpropyl | 1,1-Dimethylpropyl |

Preferred compounds of TABLE I are named as follows:
- 1: (2S)-2-({1-Oxo-5-phenyl}-pentyl-1-(3,3-dimethyl-1,2-dioxopentyl)pyrrolidine
- 2: 3,3-Dimethyl-1-[(2*S*)-2-(5-(3-pyridyl)pentanoyl)-1-pyrrolidine]-1,2-pentanedione
- 3: (2S)-2-({1-Oxo-4-phenyl}-butyl-1-(3,3-dimethyl-1,2-dioxobutyl)pyrrolidine
- 9: 2-Phenyl-1-ethyl (2*S*)-1-(3,3-dimethyl-1,2-dioxopentyl)-2-pyrrolidinecarbothioate
- 10: 2-Phenyl-1-ethyl 1-(3,3-dimethyl-1,2-dioxopentyl)-2-piperidinecarbothioate
- 11: (3-Thioindolyl) methyl (2S) -1- (3, 3-dimethyl-1, 2-dioxopentyl)-2-pyrrolidinecarbothioate
- 12: 2-Phenyl-1-ethyl (2S)-1-(2-cyclohexyl-1,2-dioxoethyl)-2-pyrrolidinecarbothioate
- 14: 2-Phenyl-1-ethyl 1-(2-phenyl-1,2-dioxoethyl)-2-piperidinecarbothioate
- 28: 2-Phenyl-1-ethyl (2S)-1-(1-cyclopentyl-1,2-dioxoethyl)-2-pyrrolidinecarbothioate
- 29: 3-Phenyl-1-propyl 1-(3,3-dimethyl-1,2-dioxobutyl)-2-piperidinecarbothioate
- 30: 3-Phenyl-1-propyl (2S)-1-(3,3-dimethyl-1,2-dioxopentyl)-2-pyrrolidinecarbothioate
- 31: 3-(3-Pyridyl)-1-propyl (2S)-1-(3,3-dimethyl-1,2-dioxopentyl)-2-pyrrolidinecarbothioate
- 32: 3-Phenyl-1-propyl (2S)-1-(2-cyclohexyl-1,2-dioxoethyl)-2-pyrrolidinecarbothioate
- 33: 4-Phenyl-1-butyl (2S)-1-(2-cyclohexyl-1,2-dioxoethyl)-2-pyrrolidinecarbothioate
- 34: 4-Phenyl-1-butyl (2S)-1-(3,3-dimethyl-1,2-dioxopentyl)-2-pyrrolidinecarbothioate
- 35: 3- (3-Pyridyl) -1-propyl (2S) -1- (2-cyclohexyl-1, 2-dioxoethyl)-2-pyrrolidinecarbothioate
- 36: 3,3-Diphenyl-1-propyl (2S)-1-(3,3-dimethyl-1,2-dioxopentyl)-2-pyrrolidinecarbothioate
- 37: 3,3-Diphenyl-1-propyl (2S)-1-(2-cyclohexyl-1,2-dioxoethyl)-2-pyrrolidinecarbothioate
- 38: 3-(*para*-Methoxyphenyl)-1-propyl (2S)-1-(3,3-dimethyl-1,2-dioxopentyl)-2-pyrrolidinecarbothioate
- 39: 4-Phenyl-1-butyl 1-(1,2-dioxo-3,3-dimethylbutyl) -2-piperidinecarbothioate
- 40: 1,5-Diphenyl-3-pentyl 1-(3,3-dimethyl-1,2-dioxopentyl)-2-piperidinecarbothioate
- 41: 1,5-Diphenyl-3-mercaptopentyl 1-(3-phenyl-1,2-dioxoethyl)-2-piperidinecarbothioate
- 42: 3-(para-Methoxyphenyl)-1-propyl 1-(1,2-dioxo-3,3-dimethylpentyl)piperidine-2-carbothioate
- 43: 3-(para-Methoxyphenyl)-1-propyl 1-(2-phenyl-1,2-dioxoethyl)piperidine-2-carbothioate
- 44: 3-(1-Naphthyl)-1-propyl 1-(3,3-dimethyl-1,2-dioxopentyl)piperidine-2-carbothioate
- 45: 3,3-Di(*para*-fluoro)phenyl-1-propyl (2S)-1-(3,3-dimethyl-1,2-dioxopentyl)-2-pyrrolidinecarbothioate
- 46: 4,4-Di(para-fluorophenyl)butyl 1-(3,3-dimethyl-2-oxopentanoyl)-2-pyrrolidinecarbothioate
- 47: 3-(1-Naphthyl)propyl (2*S*)-1-(3,3-dimethyl-2-oxopentanoyl)-2-pyrrolidinecarbothioate
- 48: 2,2-Diphenylethyl (2*S*)-1-(3,3-dimethyl-2-oxopentanoyl)tetrahydro-1H-2-pyrrolidinecarbothioate
- 49: 2,2-Diphenylethyl (2S)-1-(3,3-dimethyl-2-oxopentanoyl)-2-piperidinecarbothioate
- 50: 3,3-Diphenylpropyl 1-(3,3-dimethyl-2-oxopentanoyl)-2-piperidinecarbothioate
- 51: 3-[4-(Trifluoromethyl)phenyl]propyl (2S) -1- (3, 3-dimethyl-2-oxopentanoyl)-2-pyrrolidinecarbothioate
- 52: 3-(2-Naphthyl)propyl (2S)-1-(3,3-dimethyl-2-oxopentanoyl)-2-pyrrolidinecarbothioate
- 53: 3-(2-Naphthyl)propyl (2R, S) -1- (3, 3-dimethyl-2-oxopentanoyl)-2-piperidinecarbothioate
- 54: 3-(3-Chlorophenyl)propyl (2S)-1-(3,3-dimethyl-2-oxopentanoyl)-2-pyrrolidinecarbothioate
- 55: 3- [3- (Trifluoromethyl)phenyl]propyl (2S)-1-(3,3-dimethy1-2-oxopentanoy1)-2-pyrrolidinecarbothioate
- 56: 3-(1-Biphenyl)propyl (2S)-1-(3,3-dimethyl-2-oxopentanoyl)-2-pyrrolidinecarbothioate
- 57: 3-(2-Fluorophenyl)propyl (2S) -1- (3, 3-dimethyl-2-oxopentanoyl)-2-pyrrolidinecarbothioate
- 58: 3-(3-Fluorophenyl)propyl (2S)-1-(3,3-dimethyl-2-oxopentanoyl)-2-pyrrolidinecarbothioate
- 59: 4-Phenylbutyl 1-(3,3-dimethyl-2-oxopentanoyl)-2-piperidinecarbothioate
- 60: 3-Phenylpropyl 1-(3,3-dimethyl-2-oxopentanoyl)-2-piperidinecarbothioate
- 61: 3-(2-Chlorophenyl)propyl (2S) -1- (3, 3-dimethyl-2-oxopentanoyl)-2-pyrrolidinecarbothioate
- 62: 3-(2-Chlorophenyl)propyl 1-(3,3-dimethyl-2-oxopentanoyl)-2-piperidinecarbothioate
- 63: 3-(2-Fluorophenyl)propyl 1-(3,3-dimethyl-2-oxopentanoyl)-2-piperidinecarbothioate
- 64: 3-(3-Fluorophenyl)propyl 1-(3,3-dimethyl-2-oxopentanoyl)-2-piperidinecarbothioate
- 65: 3-(3,4-Dimethoxyphenyl)propyl (2S)-1-(3,3-dimethy1-2-oxopentanoy1)-2-pyrrolidinecarbothioate
- 66: (2S)-2-({1-Oxo-4-phenyl}-butyl-1-(2-Cyclohexyl-1,2-dioxoethyl)pyrrolidine
- 67: 2-({1-Oxo-4-phenyl}-butyl-1-(3,3-dimethyl-1,2-dioxobutyl)pyrrolidine
- 68: 2-({1-Oxo-6-phenyl}-hexyl-1-(2-Cyclohexyl-1,2-dioxoethyl)piperidine
- 69: 2-({1-Oxo-[2-{2'-phenyl}ethyl]-4-phenyl}-butyl-1-(3,3-dimethyl-1,2-dioxobutyl)piperidine
- 70: 1-{(2S)-2-[5,5-di(4-Fluorophenyl)pentanoyl]-2-pyrrolidine}-3,3-dimethyl-1,2-pentanedione
- 71: 3,3-Dimethyl-1-[2-(4-phenylpentanoyl)piperidino]-1,2-pentanedione

In another preferred embodiment of formula II, the heterocyclic thioester or ketone is selected from the group consisting of GPI 1389, of the formula 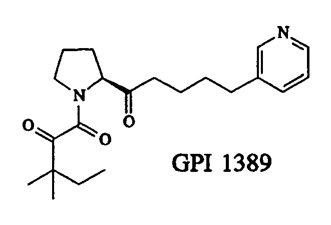 GPI 1511, of the formula 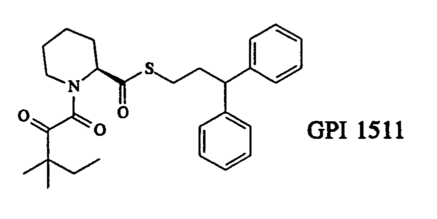 and GPI 1234, of the formula

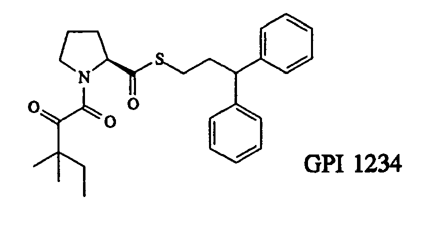

### FORMULA III

Furthermore, the heterocyclic thioester or ketone may be a compound of formula III 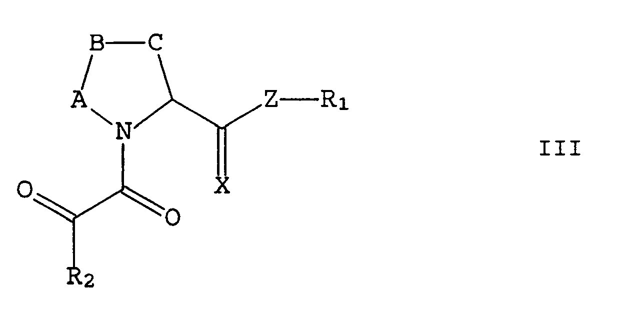 or a pharmaceutically acceptable salt, ester, or solvate thereof, wherein:
A, B, and C are independently CH₂, O, S, SO, SO₂, NH or NR₂ ;
X is O or S;
Z is S, CH₂, CHR₁ or CR₁R₃ ;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)ₙ C₁-C₆ straight or branched chain alkyl or C₂-C₆ straight or branched chain alkenyl substituted with (Ar₁)ₙ , C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁, wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxyl; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group including, but not limited to, halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Preferred compounds of formula III are presented in TABLE II.

**TABLE II**

| No. | A | B | C | X | Z | R₁ | R₂ |
|---|---|---|---|---|---|---|---|
| 72 | CH₂ | S | CH₂ | O | S | 2-phenethyl | 1,1-dimethyl-propyl |
| 73 | CH₂ | S | CH₂ | O | CH₂ | 3-phenylpropyl | 1,1-dimethyl-propyl |
| 74 | CH₂ | CH₂ | NH | O | S | 2-phenethyl | 1,1-dimethyl-propyl |
| 75 | CH₂ | S | CH₂ | S | S | 2-phenethyl | 1,1-dimethyl-propyl |

In another preferred embodiment of formula III, the heterocyclic thioester or ketone is selected from the group consisting of GPI 1389, of the formula 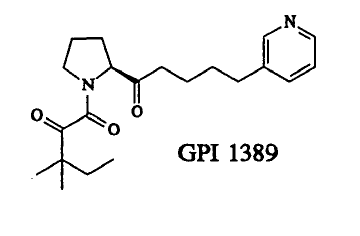 GPI 1511, of the formula 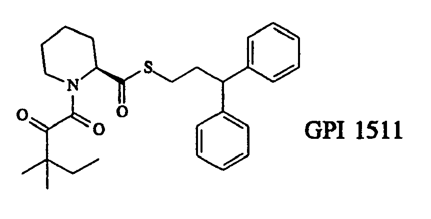 and GPI 1234, of the formula

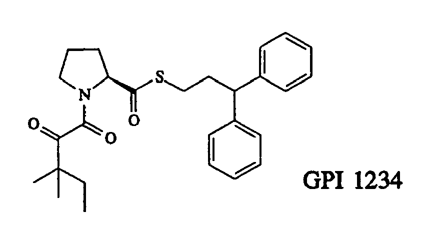

### FORMULA IV

Alternatively, the heterocyclic thioester or ketone may be a compound of formula IV 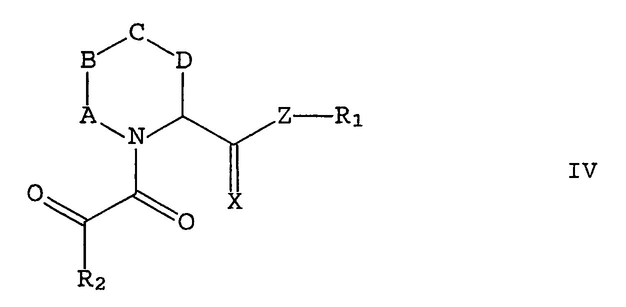 or a pharmaceutically acceptable salt, ester, or solvate thereof, wherein:
A, B, C and D are independently CH₂, O, S, SO, SO₂, NH or NR₂;
X is O or S;
Z is S, CH₂, CHR₁ or CR₁R₃ ;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)ₙ, C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁, wherein said alkyl, alkenyl, cycloalkyl or cycloalkenyl is either unsubstituted or substituted with one or more substituent(s) independently selected from the group consisting of C₃-C₈ cycloalkyl, C₁-C₄ straight or branched chain alkyl, C₂-C₄ straight or branched chain alkenyl, and hydroxyl; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group including, but not limited to, halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Preferred compounds of formula IV are presented in TABLE III.

**TABLE III**

| No. | A | B | C | D | X | Z | R₁ | R₂ |
|---|---|---|---|---|---|---|---|---|
| 76 | CH₂ | CH₂ | O | CH₂ | O | CH₂ | 3-phenylpropyl | 1,1-dimediylpropyl |
| 77 | CH₂ | CH₂ | O | CH₂ | O | S | 2-phenethyl | 1,1-dimethylpropyl |
| 78 | CH₂ | CH₂ | S | CH₂ | O | CH₂ | 3-phenylpropyl | 1,1-dinethylpropyl |
| 79 | CH₂ | CH₂ | S | CH₂ | O | S | 2-phenethyl | 1,1-dimethylpropyl |

### FORMULA V

Alternatively, the heterocyclic thioester or ketone may be a compound of formula V 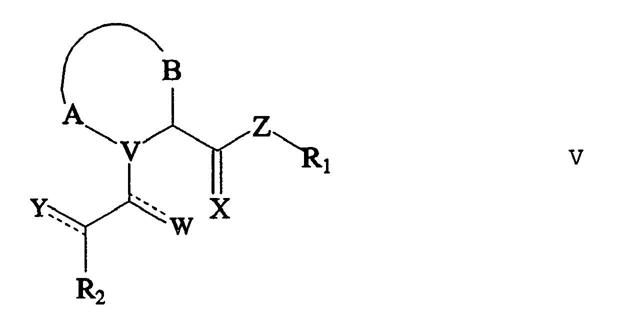 or a pharmaceutically acceptable salt, ester, or solvate thereof, wherein:
V is C, N, or S;
A and B, taken together with V and the carbon atom to which they are respectively attached, form a 5-7 membered saturated or unsaturated heterocyclic ring containing, in addition to V, one or more heteroatom(s) independently selected from the group consisting of O, S, SO, SO₂, N, NH, and NR₄;
R₄ is either C₁-C₉ straight or branched chain alkyl, C₂-C₉ straight or branched chain alkenyl, C₃-C₉ cycloalkyl, C₅-C₇ cycloalkenyl, or Ar₃, wherein R₄ is either unsubstituted or substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄;
Ar₃ and Ar₄ are independently an alicyclic or aromatic, mono-, bi- or tricyclic, carbo- or heterocyclic ring; wherein the individual ring size is 5-8 members; wherein said heterocyclic ring contains 1-6 heteroatom(s) independently selected from the group consisting of O, N, and S; and
R₁, R₂, W, X, Y, and Z are as defined in Formula I above.

All the compounds of Formulas I-V possess asymmetric centers and thus can be produced as mixtures of stereoisomers or as individual R- and S-stereoisomers. The individual stereoisomers may be obtained by using an optically active starting material, by resolving a racemic or non-racemic mixture of an intermediate at some appropriate stage of the synthesis, or by resolving the compounds of Formulas I-V. It is understood that the compounds of Formulas I-V encompass individual stereoisomers as well as mixtures (racemic and non-racemic) of stereoisomers. Preferably, S-stereoisomers are used in the pharmeceutical compositions and methods of the present invention.

### Affinity for FKBP12

The compounds used in the present invention have an affinity for the FK506 binding protein, particularly FKBP12. The inhibition of the prolyl peptidyl *cis-trans* isomerase activity of FKBP may be measured as an indicator of this affinity.

### Kᵢ Test Procedure

Inhibition of the peptidyl-prolyl isomerase (rotamase) activity of the compounds used in the inventive methods and pharmaceutical compositions can be evaluated by known methods described in the literature (Harding et al., Nature, 1989, 341:758-760; Holt et al. *J. Am. Chem. Soc.,* 115:9923-9938). These values are obtained as apparent K₁'s and are presented for representative compounds in TABLE IV.

The *cis-trans* isomerization of an alanine-proline bond in a model substrate, N-succinyl-Ala-Ala-Pro-Phe-p-nitroanilide, is monitored spectrophotometrically in a chymotrypsin-coupled assay, which releases paranitroanilide from the trans form of the substrate. The inhibition of this reaction caused by the addition of different concentrations of inhibitor is determined, and the data is analyzed as a change in first-order rate constant as a function of inhibitor concentration to yield the apparent Kᵢ values.

In a plastic cuvette are added 950 mL of ice cold assay buffer (25 mM HEPES, pH 7.8, 100 mM NaCl), 10 mL of FKBP (2.5 mM in 10 mM Tris-Cl pH 7.5, 100 mM NaCl, 1 mM dithiothreitol), 25 mL of chymotrypsin (50 mg/ml in 1 mM HCl) and 10 mL of test compound at various concentrations in dimethyl sulfoxide. The reaction is initiated by the addition of 5 mL of substrate (succinyl-Ala-Phe-Pro-Phe-para-nitroanilide, 5 mg/mL in 2.35 mM LiCl in trifluoroethanol).

The absorbance at 390 nm versus time is monitored for 90 seconds using a spectrophotometer and the rate constants are determined from the absorbance versus time data files.

**TABLE IV**

| *In Vitro* Test Results - Formulas I to V | |
|---|---|
| Compound | Kᵢ (nM) |
| 1 | 31 |
| 2 | 210 |
| 3 | 85 |
| 9 | 104 |
| 10 | 12 |
| 11 | 299 |
| 12 | 442 |
| 14 | 313 |
| 28 | 108 |
| 29 | 59 |
| 30 | 11 |
| 31 | 8.7 |
| 32 | 362 |
| 33 | 1698 |
| 34 | 34 |
| 35 | 62 |
| 36 | 7 |
| 37 | 68 |
| 38 | 8.9 |
| 39 | 347 |
| 40 | 1226 |
| 41 | 366 |
| 42 | 28 |
| 43 | 259 |
| 44 | 188 |
| 45 | 31 |
| 46 | 757 |
| 47 | 21 |
| 48 | 127 |
| 49 | 1334 |
| 50 | 55 |
| 51 | 33 |
| 52 | 6 |
| 53 | 261 |
| 54 | 37 |
| 55 | 30 |
| 56 | 880 |
| 57 | 57 |
| 58 | 79 |
| 59 | 962 |
| 60 | 90 |
| 61 | 139 |
| 62 | 196 |
| 63 | 82 |
| 64 | 163 |
| 65 | 68 |
| 66 | 306 |
| 67 | 177 |
| 68 | 284 |
| 69 | 49 |
| 70 | 457 |
| 71 | 788 |

### Route of Administration

To effectively treat alopecia or promote hair growth, the compounds used in the inventive methods and pharmaceutical compositions must readily affect the targeted areas. For these purposes, the compounds are preferably administered topically to the skin.

For topical application to the skin, the compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water. Alternatively, the compounds can be formulated into suitable lotions or creams containing the active compound suspended or dissolved in, for example, a mixture of one or more of the following: mineral oil, sorbitan monostearate, polysorbate 60, cetyl ester wax, cetearyl alcohol,' 2-octyldodecanol, benzyl alcohol and water.

Other routes of administration known in the pharmaceutical art are also contemplated by this invention.

### Dosage

Dosage levels on the order of about 0.1 mg to about 10,000 mg of the active ingredient compound are useful in the treatment of the above conditions, with preferred levels of about 0.1 mg to about 1,000 mg. The specific dose level for any particular patient will vary depending upon a variety of factors, including the activity of the specific compound employed; the age, body weight, general health, sex and diet of the patient; the time of administration; the rate of excretion; drug combination; the severity of the particular disease being treated; and the form of administration. Typically, in vitro dosage-effect results provide useful guidance on the proper doses for patient administration. Studies in animal models are also helpful. The considerations for determining the proper dose levels are well known in the art.

The compounds can be administered with other hair revitalizing agents. Specific dose levels for the other hair revitalizing agents will depend upon the factors previously stated and the effectiveness of the drug combination.

### EXAMPLES

The following examples are illustrative of the present invention and are not intended to be limitations thereon. Unless otherwise indicated, all percentages are based upon 100% by weight of the final composition.

### Example 1

### Synthesis of (2S)({1-oxo-5-phenyl}-pentyl-1-(3,3-dimethyl-1,2-dioxopentyl)pyrrolidine (Compound 1)

### (2S)-2-(1-oxo-4 phenyl)butyl-N-benzylpyrrolidine

1-chloro-4-phenylbutane (1.78 g; 10.5 mmol) in 20 mL of THF was added to 0.24 g (10 mmol) of magnesium turnings in 50 mL of refluxing THF. After the addition was complete, the mixture was refluxed for an additional 5 hours, and then added slowly to a refluxing solution of N-benzyl-L-proline ethyl ester (2.30 g (10 mmol) in 100 mL of THF. After 2 hours of further reflux, the mixture was cooled and treated with 5 mL of 2 N HCl. The reaction mixture was diluted with ether (100 mL) and washed with saturated NaHCO₃, water and brine. The organic phase was dried, concentrated and chromatographed, eluting with 5:1 CH₂Cl₂:EtOAc to obtain 2.05 g (64%) of the ketone as an oil. ¹H NMR (CDCl₃; 300 MHz): 1.49-2.18 (m, 8H); 2.32-2.46 (m, 1H); 2.56-2.65 (m, 2H); 2.97-3.06 (m, 1H); 3.17-3.34 (m, 1H); 3.44-3.62 (m, 1H); 4.02-4.23 (m, 2H); 7.01-7.44 (m, 10H).

### (2S)-2-(l-oxo-4-r)phenyl)butylpyrrolidine

The ketone compound (500 mg) and palladium hydroxide (20% on carbon, 50 mg) was hydrogenated at 40 psi in a Paar shaker overnight. The catalyst was removed by filtration and the solvent was removed in vacuo. The free amine was obtained as a yellow oil (230 mg; 100%). ¹H NMR (CDCl₃; 300 MHz): 1.75-2.34 (m, 10H); 2.55 (m, 2H); 2.95 (dm, 1H); 3.45-3.95 (m, 1H); 4.05 (m, 1H); 7.37 (m, 5H).

### (2S)-2-(1-oxo-4-phenyl)butyl-1-(1,2-dioxo-2-methoxyethyl)pyrrolidine

To a solution of (2S) -2- (1-oxo-4-phenyl)butylpyrrolidine (230 mg; 1.0 mmol) in CH₂Cl₂(20 mL) at 0°C was added dropwise methyloxalyl chloride (135 mg; 1.1 mmol). After stirring at 0°C for 3 hours, the reaction was quenched with saturated NH₄Cl and the organic phase was washed with water and brine and dried and concentrated. The crude residue was purified on a silica gel column, eluting with 20:1 CH₂Cl₂:EtOAc to obtain 300 mg of the oxamate as a clear oil (98%). ¹H NMR (CDCl₃; 300 MHz): 1.68 (m, 4H); 1.91-2.38 (m, 4H); 2.64 (t, 2H); 3.66-3.80 (m, 2H); 3.77, 3.85 (s, 3H total); 4.16 (m, 2H); 4.90 (m, 1H); 7.16 (m, 3H); 7.27 (m, 2H).

### (2S)-2-({1-oxo-5-nhenyl}-pentyl-1-(3,3-dimethyl-1,2-dioxopentyl)pyrrolidine (1)

To a solution of the oxamate above (250 mg; 0.79 mmol) in anhydrous ether (15 mL), cooled to - 78°C, was added 1,1-dimethylpropyl-magnesium chloride (0.8 mL of a 1.0 M solution in ether; 0.8 mmol). After stirring the resulting mixture at -78°C for 2 hours, the reaction was quenched by the addition of 2 mL of saturated NH₄Cl, followed by 100 mL of EtOAc. The organic phase was washed with brine, dried, concentrated, and purified on a silica gel column, eluting with 50:1 CH₂Cl₂:EtOAc. Compound 1 was obtained as a clear oil, 120 mg. ¹H NMR (CDCl₃, 300 MHz): δ 0.87 (t, 3H, J = 7.5); 1.22 (s, 3H); 1.25 (s, 3H); 1.67 (m, 4H); 1.70-2.33 (m, 6H); 2.61 (t, 2H, J = 7.1); 3.52 (m, 2H); 4.17 (t, 2H, J = 6.2); 4.52 (m, 1H); 7.16-7.49 (m, 5H). Analysis calculated for C₂₂H₃₁NO₃ - H₂O: C, 70.37; H, 8.86; N, 3.73. Found: 70.48; H, 8.35; N, 3.69.

### Example 2

### Synthesis of 2-phenyl-1-ethyl 1-(3,3-dimethyl-1,2-dioxopentyl)-2-piperidinecarbothioate (10)

### Methyl (2S)-1-(1,2-dioxo-2-methoxyethyl)-2-pyrrolidinecarboxylate

A solution of L-proline methyl ester hydrochloride (3.08 g; 18.60 mmol) in dry methylene chloride was cooled to 0°C and treated with triethylamine (3.92 g; 38.74 mmol; 2.1 eq). After stirring the formed slurry under a nitrogen atmosphere for 15 min, a solution of methyl oxalyl chloride (3.20 g; 26.12 mmol) in methylene chloride (45 mL) was added dropwise. The resulting mixture was stirred at 0°C for 1,5 hour. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of *cis-trans* amide rotamers; data for trans rotamer given. ¹H NMR (CDCl₃): δ 1.93 (dm, 2H); 2.17 (m, 2H); 3.62 (m, 2H); 3.71 (s, 3H); 3.79, 3.84 (s, 3H total); 4.86 (dd, 1H, j = 8.4, 3.3).

### Methyl(2S)-1-(1,2-dioxo-3,3-dimethylpentyl)-2-Pyrrolidinecarboxylate

A solution of methyl (2S)-1-(1,2-dioxo-2-methoxyethyl)-2-pyrrolidinecarboxylate (2.35 g; 10.90 mmol) in 30 mL of tetrahydrofuran (THF) was cooled to -78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): δ 0.88 (t, 3H); 1.22, 1.26 (s, 3H each); 1.75(dm, 2H); 1.87-2.10 (m, 3H); 2.23 (m, 1H); 3.54 (m, 2H); 3.76 (s, 3H); 4.52 (dm, 1H, J = 8.4, 3.4).

### (2S)-1-(1,2-dioxo-3,3-dimethylpentyl)-2-pyrrolidinecarboxylic acid

A mixture of methyl (2S)-1-(1,2-dioxo-3,3-dimethylpentyl)-2-pyrrolidinecarboxylate (2.10 g; 8.23 mmol), 1 N LiOH (15 mL), and methanol (50 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃) : δ 0.87 (t, 3H); 1.22, 1.25 (s, 3H each); 1.77 (dm, 2H); 2.02 (m, 2H); 2.17 (m, 1H); 2.25 (m, 1H); 3.53 (dd, 2H, j = 10.4, 7.3); 4.55 (dd, 1H, j = 8.6, 4.1).

### 2-phenyl-1-ethyl 1-(3,3-dimethyl-1,2-dioxopentyl)-2-piperidinecarbothioate (10)

To a solution of (2S)-1-(1,2-dioxo-3,3-dimethylpentyl)-2-pyrrolidinecarboxylic acid (241 mg; 1.0 mmol) in CH₂Cl₂ (10 mL) was added dicyclohexylcarbo-diimide (226 mg; 1.1 mmol). After stirring the resulting mixture for 5 minutes, the solution was cooled to 0°C and treated with a solution of phenyl mercaptan (138 mg; 1.0 mmol) and 4-dimethylaminopyridine (6 mg) in 5 ml of CH₂Cl₂. The mixture was allowed to warm to room temperature with stirring overnight. The solids were removed by filtration and the filtrate was concentrated in vacuo; the crude residue was purified by flash chromatography (10:1 hexane:EtOAc) to obtain 302 mg (84%) of compound 10 as an oil. ¹H NMR (CDCl₃, 300 MHz): δ 0.85 (t, 3H, J = 7.5); 1.29 (s, 3H); 1.31 (s, 3H); 1.70-2.32 (m, 6H) ; 2.92 (t, 2H, j = 7.4) ; 3.22 (t, 2H, J = 7.4) ; 3.58 (m, 2H); 4.72 (m, 1H); 7.23-7.34 (m, 5H). Analysis calculated for C₂₀H₂₇NO₃S - 0.4 H₂O: C, 65.15; H, 7.60; N, 3.80. Found: C, 65.41; H, 7.49; N, 3.72.

### Example 3

### Synthesis of 2-phenyl-1-ethyl (2S)-1-(3,3-dimethyl-1,2-dioxonentyl)-2-pyrrolidinecarbothioate (9)

### Methyl 1-(1,2-dioxo-2-methoxyethyl)-2-piperidine-carboxylate

A solution of methyl pipecolate hydrochloride (8.50 g; 47.31 mmol) in dry methylene chloride (100 mL) was cooled to 0°C and treated with triethylamine (10.5 g; 103 mmol; 2.1 eq). After stirring the formed slurry under a nitrogen atmosphere for 15 minutes, a solution of methyl oxalyl chloride (8.50 g; 69.4 mmol) in methylene chloride (75 mL) was added dropwise. The resulting mixture was stirred at 0°C for 1,5 hours. After filtering to remove solids, the organic phase was washed with water, dried over MgSO₄ and concentrated. The crude residue was purified on a silica gel column, eluting with 50% ethyl acetate in hexane, to obtain 9.34 g (86%) of the product as a reddish oil. Mixture of *cis-trans* amide rotamers; data for trans rotamer given. ¹H NMR (CDCl₃) : δ 1.22-1.45 (m, 2H); 1.67-1.78 (m, 3H); 2.29 (m, 1H); 3.33 (m, 1H); 3.55 (m, 1H); 3.76 (s, 3H); 3.85, 3.87 (s, 3H total); 4.52 (dd, 1H).

### Methyl 1-(1,2-dioxo-3,3-dimethylpentyl)-2-piperidine-carboxylate

A solution of methyl 1-(1,2-dioxo-2-methoxyethyl)-2-piperidinecarboxylate (3.80 g; 16.57 mmol) in 75 mL of tetrahydrofuran (THF) was cooled to -78°C and treated with 20.7 mL of a 1.0 M solution of 1,1-dimethyl-propylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 3.32 g (74%) of the oxamate as a colorless oil. ¹H NMR (CDCl₃): δ 0.88 (t, 3H); 1.21, 1.25 (s, 3H each); 1.35-1.80 (m, 7H); 2.35 (m, 1H); 3.24 (m, 1H); 3.41 (m, 1H); 3.76 (s, 3H); 5.32 (d, 1H).

### 1-(1,2-dioxo-3,3-dimethylpentyl)-2-piberidine-carboxylic acid

A mixture of methyl 1-(1,2-dioxo-3,3-dimethylpentyl)-2-piperidinecarboxylate (3.30 g; 12.25 mmol), 1 N LiOH (15 mL), and methanol (60 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 2.80 g (87%) of snow-white solid which did not require further purification. ¹H NMR (CDCl₃) : δ 0.89 (t, 3H); 1.21, 1.24 (s, 3H each); 1.42-1.85 (m, 7H); 2.35 (m, 1H); 3.22 (d, 1H); 3.42(m, 1H); 5.31 (d, 1H).

### 2-phenyl-1-ethyl (2S)-1-(3,3-dimethyl-1,2-dioxopentyl)-2-pyrrolidinecarbothioate (9)

To a solution of 1-(1,2-dioxo-3,3-dimethylpentyl)-2-piperidine-carboxylic acid (255 mg; 1.0 mmol) in CH₂Cl₂ (10 mL) was added dicyclohexylcarbodiimide (226 mg; 1.1 mmol). After stirring the resulting mixture for 5 minutes, the solution was cooled to 0°C and treated with a solution of phenyl mercaptan (138 mg; 1.0 mmol) and 4-dimethylaminopyridine (6 mg) in 5 ml of CH₂Cl₂. The mixture was allowed to warm to room temperature with stirring overnight. The solids were removed by filtration and the filtrate was concentrated in vacuo; the crude residue was purified by flash chromatography (10:1 hexane:EtOAc) to obtain 300 mg (80%) of compound 9 as an oil. ¹H NMR (CDCl₃, 300 MHz) : d 0.94 (t, 3H, = 7.5); 1.27 (s, 3H); 1.30 (s, 3H); 1.34-1.88 (m, 7H); 2.45 (m, 1H); 2.90 (t, 2H, J = 7.7); 3.26 (t, 2H, J = 7.7); 3.27 (m, 1H); 3.38 (m, 1H); 5.34 (m, 1H) ; 7.24-7.36 (m, 5H). Analysis calculated for C₂₁H₂₉NO₃S: C, 67.17; H, 7.78; N, 3.73. Found: C, 67.02; H, 7.83; N, 3.78.

### Example 4

### In Vivo Hair Generation Tests With C57 Black 6 Mice

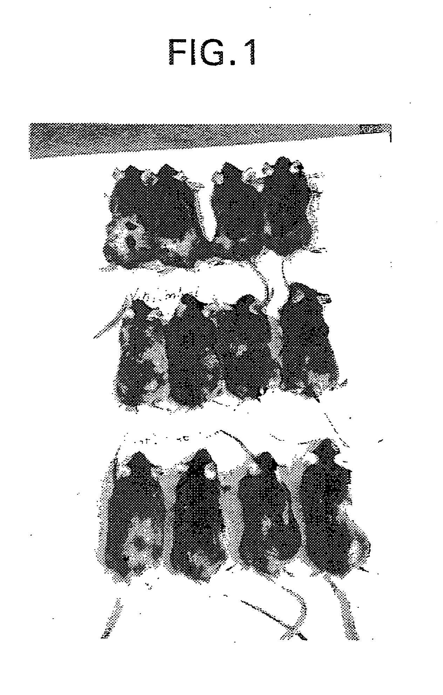

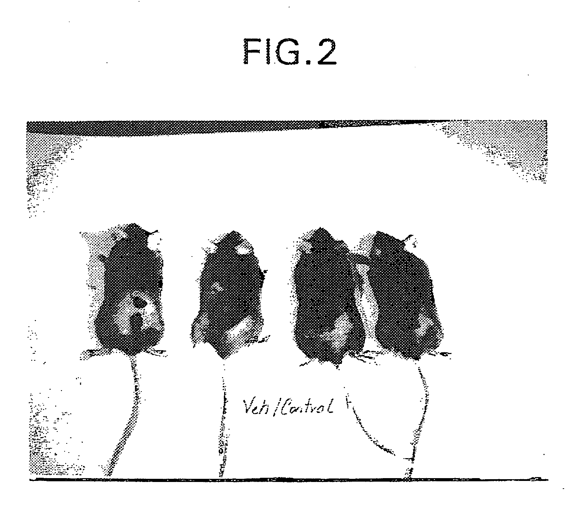

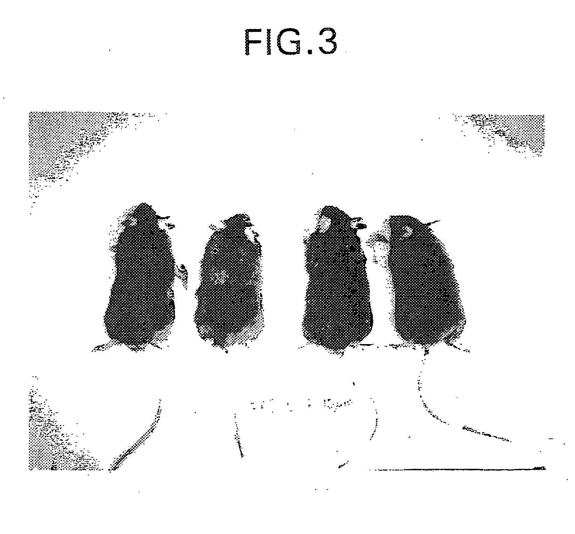

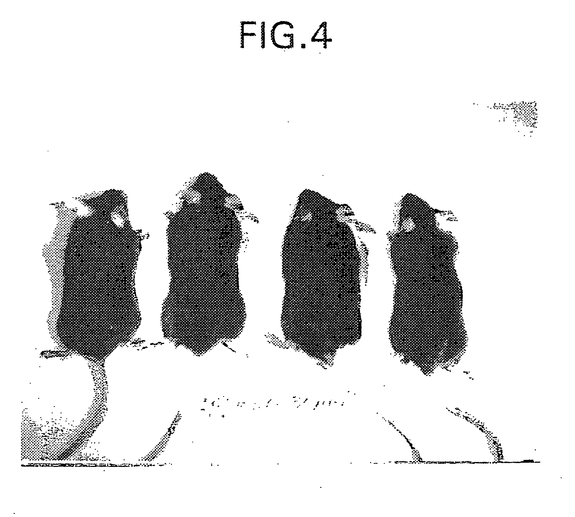

Experiment A: C57 black 6 mice were used to demonstrate the hair revitalizing properties of a low molecular weight, small molecule non-immunosuppressive neuroimmunophilin FKBP ligand, GPI 1046, which is related to heterocyclic thioesters and ketones. Referring now to FIGS. 1 and 2 of the drawings, C57 black 6 mice, approximately 7 weeks old, had an area of about 2 inches by 2 inches on their hindquarters shaved to remove all existing hair. Care was taken not to nick or cause abrasion to the underlaying dermal layers. The animals were in anagen growth phase, as indicated by the pinkish color of the skin. Referring now to FIGS. 2, 3 and 4, four animals per group were treated by topical administration with 20% propylene glycol vehicle (FIG. 2), 10 µM GPI 1046 (FIG. 3) or 30 µM GPI 1046 (FIG. 4) dissolved in the vehicle. The animals were treated with vehicle or GPI 1046 every 48 hours (3 applications total over the course of 5 days) and the hair growth was allowed to proceed for 6 weeks. Hair growth was quantitated by the percent of shaved area covered by new hair growth during this time period.

FIG. 2 shows that animals treated with vehicle exhibited only a small amount of hair growth in patches or tufts, with less than 3% of the shaved area covered with new growth. In contrast, FIG. 3 shows that animals treated with 10 *µ*M GPI 1046 exhibited dramatic hair growth, covering greater than 90% of the shaved area in all animals. Further, FIG. 4 shows that mice treated with 30 µM GPI 1046 exhibited essentially complete hair regrowth and their shaved areas were indistinguishable from unshaven C57 black 6 mice.

Experiment B: C57 Black 6 mice were used to demonstrate the hair revitalizing properties of a variety of low molecular weight, small molecule, non-immunosuppressive neuroimmunophilin FKBP ligands, including GPI 1389, 1511, and 1234. C57 Black 6 mice, 55 to 75 days old, had an area of about 2 inches by 2 inches on their hindquarters shaved to remove all existing hair. Care was taken not to nick or cause abrasion to the underlying dermal layers. The animals were in anagen growth phase when shaved. Five animals per group were treated by topical administration with a vehicle, FK506, or one of the low molecular weight, small molecule, non-immunosuppressive neuroimmunophilin FKBP ligands (GPI 1605, 1046, 1312, 1572, 1389, 1511, and 1234) at a concentration of one micromole per milliliter to the shaved area. The animals were treated three times per week, and hair growth was evaluated 14 days after initiation of treatment. Hair growth was quantitated by the percent of shaved area covered by new hair growth, as scored by a blinded observer, on a scale of 0 (no growth) to five (complete hair regrowth in shaved area).

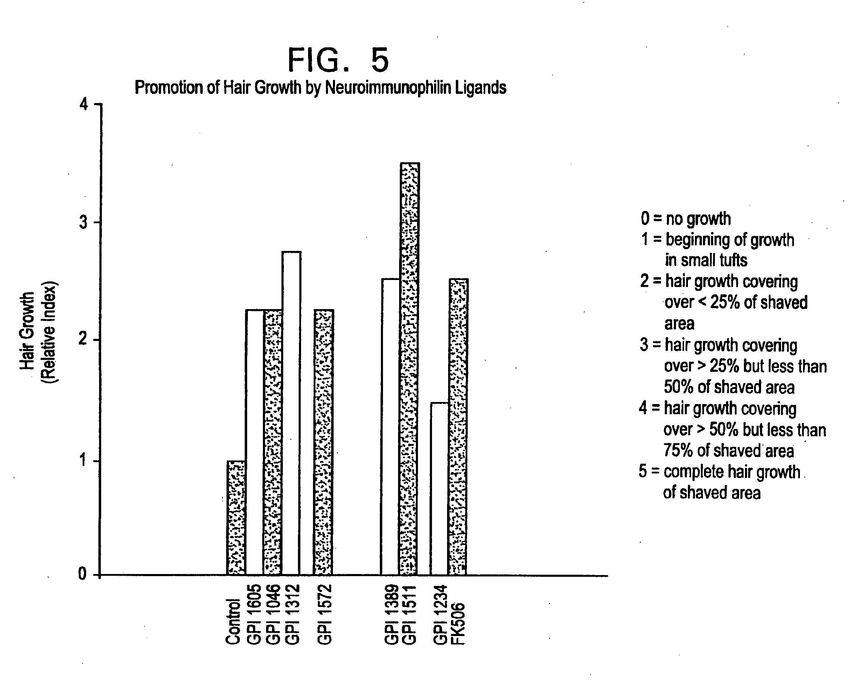

Figure 5 shows that after 14 days, the animals treated with vehicle exhibited the beginning of growth in small tufts. In contrast, animals treated with one of the low molecular weight, small molecule, non-immunosuppressive neuroimmunophilin FKBP ligands, including GPI 1389, 1511, and 1234, exhibited dramatic hair growth.

### Example 5

A lotion comprising the following composition may be prepared.

| | (%) |
|---|---|
| 95% Ethanol | 80.0 |
| a heterocyclic thioester or ketone as defined above | 10.0 |
| α-Tocopherol acetate | 0.01 |
| Ethylene oxide (40 mole) adducts of hardened castor oil | 0.5 |
| purified water | 9.0 |
| perfume and dye | q.s. |

Into 95% ethanol are added a heterocyclic thioester or ketone, α-tocopherol acetate, ethylene oxide (40 mole) adducts of hardened castor oil, perfume and a dye. The resulting mixture is stirred and dissolved, and purified water is added to the mixture to obtain a transparent liquid lotion.

5 ml of the lotion may be applied once or twice per day to a site having marked baldness or alopecia.

### Example 6

A lotion comprising the following composition shown may be prepared.

| | (%) |
|---|---|
| 95% Ethanol | 80.0 |
| a heterocyclic thioester or ketone as defined above | 0.005 |
| Hinokitol | 0.01 |
| Ethylene oxide (40 mole) adducts of hardened castor oil | 0.5 |
| Purified water | 19.0 |
| Perfume and dye | q.s. |

Into 95% ethanol are added a heterocyclic thioester or ketone, hinokitol, ethylene oxide (40 mole) adducts of hardened castor oil, perfume, and a dye. The resulting mixture is stirred, and purified water is added to the mixture to obtain a transparent liquid lotion.

The lotion may be applied by spraying once to 4 times per day to a site having marked baldness or alopecia.

### Example 7

An emulsion may be prepared from A phase and B phase having the following compositions.

| **(A phase)** | (%) |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitan monooleate | 1.0 |
| a heterocyclic thioester or ketone as defined above | 0.01 |

| **(B phase)** | (%) |
|---|---|
| Glycerine | 10.0 |
| Purified water | 69.0 |
| Perfume, dye, and preservative | q.s. |

The A phase and the B phase are respectively heated and melted and maintained at 80°c. Both phases are then mixed and cooled under stirring to normal temperature to obtain an emulsion.

The emulsion may be applied by spraying once to four times per day to a site having marked baldness or alopecia.

### Example 8

A cream may be prepared from A phase and B phase having the following compositions.

| **(A Phase)** | (%) |
|---|---|
| Fluid paraffin | 5.0 |
| Cetostearyl alcohol | 5.5 |
| Petrolatum | 5.5 |
| Glycerine monostearate | 33.0 |
| Polyoxyethylene (20 mole) 2-octyldodecyl ether | 3.0 |
| Propylparaben | 0.3 |

| **(B Phase)** | (%) |
|---|---|
| a heterocyclic thioester or ketone as defined above | 0.8 |
| Glycerine | 7.0 |
| Dipropylene glycol | 20.0 |
| Polyethylene glycol 4000 | 5.0 |
| Sodium Hexametaphosphate | 0.005 |
| Purified water | 44.895 |

The A phase is heated and melted, and maintained at 70°c. The B phase is added into the A phase and the mixture is stirred to obtain an emulsion. The emulsion is then cooled to obtain a cream.

The cream may be applied once to 4 times per day to a site having marked baldness or alopecia.

### Example 9

A liquid comprising the following composition may be prepared.

| | (%) |
|---|---|
| Polyoxyethylene butyl ether | 20.0 |
| Ethanol | 50.0 |
| a heterocyclic thioester or ketone as defined above | 0.001 |
| Propylene glycol | 5.0 |
| Polyoxyethylene hardened castor oil derivative (ethylene oxide 80 mole adducts) | 0.4 |
| Perfume | q.s. |
| Purified water | q.s. |

Into ethanol are added polyoxypropylene butyl ether, propylene glycol, polyoxyethylene hardened castor oil, a heterocyclic thioester or ketone, and perfume. The resulting mixture is stirred, and purified water is added to the mixture to obtain a liquid.

The liquid may be applied once to 4 times per day to a site having marked baldness or alopecia.

### Example 10

A shampoo comprising the following composition may be prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| a heterocyclic thioester or ketone as defined above | 5.0 |
| Ethanol | 2.0 |
| Perfume | 0.3 |
| Purified water | 69.7 |

Into 69.7 of purified water are added 5.0 g of sodium laurylsulfate, 5.0 g of triethanolamine laurylsulfate, 6.0 g of betaine lauryldimethylaminoacetate. Then a mixture obtained by adding 5.0 g of a heterocyclic thioester or ketone, 5.0 g of polyethylene glycol, and 2.0 g of ethylene glycol distearate to 2.0 g of ethanol, followed by stirring, and 0.3 g of perfume are successively added. The resulting mixture is heated and subsequently cooled to obtain a shampoo.

The shampoo may be used on the scalp once or twice per day.

### Example 11

A patient is suffering from alopecia senilis. A heterocyclic thioester or ketone as identified above, or a pharmaceutical composition comprising the same, may be administered to the patient. Increased hair growth is expected to occur following treatment.

### Example 12

A patient is suffering from male pattern alopecia. A heterocyclic thioester or ketone as identified above, or a pharmaceutical composition comprising the same, may be administered to the patient. Increased hair growth is expected to occur following treatment.

### Example 13

A patient is suffering from alopecia areata. A heterocyclic thioester or ketone as identified above, or a pharmaceutical composition comprising the same, may be administered to the patient. Increased hair growth is expected to occur following treatment.

### Example 14

A patient is suffering from hair loss caused by skin lesions. A heterocyclic thioester or ketone as identified above, or a pharmaceutical composition comprising the same, may be administered to the patient. Increased hair growth is expected to occur following treatment.

### Example 15

A patient is suffering from hair loss caused by tumors. A heterocyclic thioester or ketone as identified above, or a pharmaceutical composition comprising the same, may be administered to the patient. Increased hair growth is expected to occur following treatment.

### Example 16

A patient is suffering from hair loss caused by a systematic disorder, such as a nutritional disorder or an internal secretion disorder. A heterocyclic thioester or ketone as identified above, or a pharmaceutical composition comprising the same, may be administered to the patient. Increased hair growth is expected to occur following treatment.

### Example 17

A patient is suffering from hair loss caused by chemotherapy. A heterocyclic thioester or ketone as identified above, or a pharmaceutical composition comprising the same, may be administered to the patient. Increased hair growth is expected to occur following treatment.

### Example 18

A patient is suffering from hair loss caused by radiation. A heterocyclic thioester or ketone as identified above, or a pharmaceutical composition comprising the same, may be administered to the patient. Increased hair growth is expected to occur following treatment.

## Claims

1. Use of a compound for the preparation of a pharmaceutical composition or medicament for treating alopecia or promoting hair growth in an animal in need thereof, wherein said compound is of formula V 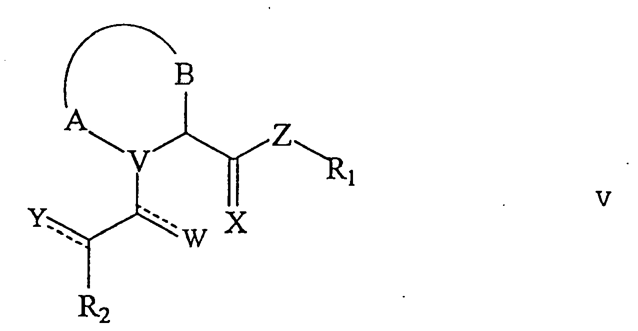 or a pharmaceutically acceptable salt, ester, or solvate thereof, wherein:
V is C, N, or S;
A and B, taken together with V and the carbon atom to which they are respectively attached, form a 5-7 membered saturated or unsaturated heterocyclic ring containing one or more heteroatom(s) independently selected from the group consisting of O, S, SO, SO₂, N, NH, and NR₄;
R₄ is either C₁-C₉ straight or branched chain alkyl, C₂-C₉ straight or branched chain alkenyl, C₃-C₉ cycloalkyl, C₅-C₇ cycloalkenyl, or Ar₃,
wherein R₄ is either unsubstituted or substituted with one or more substituent(s) independently selected from the group consisting of halo, haloalkyl, carbonyl, carboxy, hydroxy, nitro, trifluoromethyl, C₁₋C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄ ;
Ar₃ and Ar₄ are independently an alicyclic or aromatic, mono-, bi- or tricyclic, carbo- or heterocyclic ring,
wherein the individual ring size is 5-8 members,
and wherein said heterocyclic ring contains 1-6 heteroatom(s) independently selected from the group consisting of O, N, and S;
X is either O or S;
Z is either S, CH₂, CHR₁ or CR₁R₃;
W and Y are independently O, S, CH₂ or H₂;
R₁ and R₃ are independently C₁-C₆ straight or branched chain alkyl or C₂-C₆ straight or branched chain alkenyl,
wherein said alkyl or alkenyl is substituted with one or more substituent (s) independently selected from the group consisting of (Ar₁)_{n'} C₁-C₆ straight or branched chain alkyl or C₂₋C₆ straight or branched chain alkenyl substituted with (Ar₁)ₙ, C₃-C₈ cycloalkyl, C₁₋C₆ straight or branched chain alkyl or C₂-C₆ straight or branched chain alkenyl substituted with C₃-C₈ cycloalkyl, and Ar₂; n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁,
wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring,
wherein said ring is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino,
wherein the individual ring size is 5-8 members,
and wherein the heterocyclic ring has 1-6 heteroatom(s) independently selected from the group consisting of O, N, and S.

2. The use of claim 1, wherein the compound is of formula I 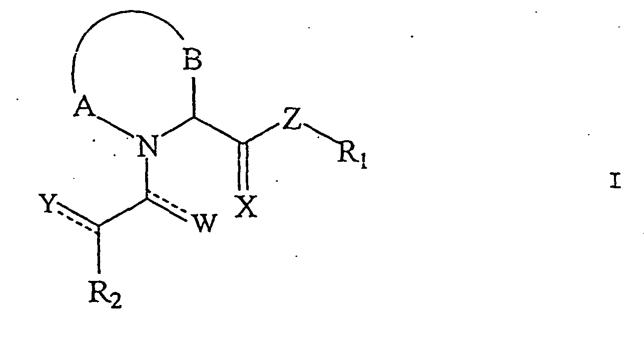 or a pharmaceutically acceptable salt, ester, or solvate thereof, wherein:
A and B, taken together with the nitrogen and carbon atoms to which they are respectively attached, form a 5-7 membered saturated or unsaturated heterocyclic ring containing one or more additional O, S, SO, SO₂, N, NH, or NR₂ heteroatom (s) ;
X is either O or S;
Z is either S, CH₂, CHR₁ or CR₁R₃;
W and Y are independently O, S, CH₂ or H₂;
R₁ and R₃ are independently C₁-C₆ straight or branched chain alkyl or C₂-C₆ straight or branched chain alkenyl,
wherein said alkyl or alkenyl is substituted with one or more substituent (s) independently selected from the group consisting of (Ar₁)ₙ, C₁-C₆ straight or branched chain alkyl or C₂₋C₆ straight or branched chain alkenyl substituted with (Ar₁)ₙ, C₃-C₈ cycloalkyl, C₁₋C₆ straight or branched chain alkyl or C₂-C₆ straight or branched chain alkenyl substituted with C₃-C₈ cycloalkyl, and Ar₂; n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁,
wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring,
wherein said ring is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino,
wherein the individual ring size is 5-8 members,
and wherein the heterocyclic ring contains 1-6 heteroatom(s) independently selected from the group consisting of O, N, and S.

3. The use of claim 2, wherein the mono- or bicyclic, carbo- or heterocyclic ring is selected from the group consisting of naphthyl, indolyl, furyl, thiazolyl, thienyl, pyridyl, quinolinyl, isoquinolinyl, fluorenyl, and phenyl.

4. The use of claim 1, wherein the compound is of formula II 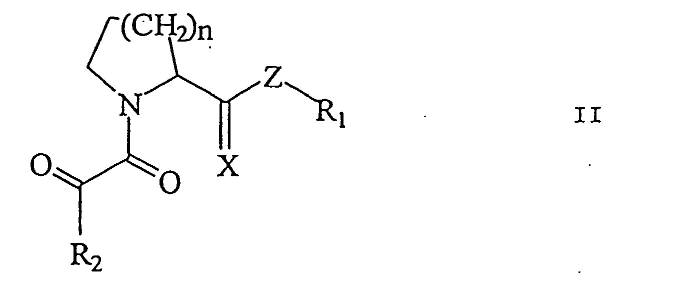 or a pharmaceutically acceptable salt, ester, or solvate thereof, wherein:
n is 1 or 2;
X is O or S;
Z is selected from the group consisting of S, CH₂, CHR₁, and CR₁R₃;
R₁ and R₃ are independently selected from the group consisting of C₁-C₅ straight or branched chain alkyl, C₂-C₅ straight or branched chain alkenyl, and Ar₁,
wherein said R₁ is unsubstituted or substituted with one or more substituent(s) independently selected from the group consisting of halo, nitro, C₁-C₆ straight or branched chain alkyl, C₂-C₆ straight or branched chain alkenyl, hydroxy, C₁-C₄ alkoxy, C₂-C₄ alkenyloxy, phenoxy, benzyloxy, amino, and Ar₁;
R₂ is selected from the group consisting of C₁-C₉ straight or branched chain alkyl, C₂-C₉ straight or branched chain alkenyl, C₃-C₈ cycloalkyl, C₅-C₇ cycloalkenyl, and Ar₁; and
Ar₁ is phenyl, benzyl, pyridyl, fluorenyl, thioindolyl or naphthyl,
wherein said Ar₁ is unsubstituted or substituted with one or more substituent(s) independently selected from the group consisting of halo, trifluoromethyl, hydroxy, nitro, C₁-C₆ straight or branched chain alkyl, C₂-C₆ straight or branched chain alkenyl, C₁₋C₄ alkoxy, C₂-C₄ alkenyloxy, phenoxy, benzyloxy, and amino.

5. The use of claim 4, wherein:
n is 1; and
X is O.

6. The use of claim 5, wherein Z is CH₂.

7. The use of claim 6, wherein the compound is selected from the group consisting of:
(2*S*) -2- ({1-Oxo-5-ph8nyl)-pentyl-1- (3, 3-dimethyl-1,2-dioxopentyl) pyrrolidine;
3,3-Dimethyl-1-[(2*S*)-2-(5-(3-pyridyl)pentanoyl)-1-pyrrolidine]-1,2-pentanedione;
(2*S*)-2-({1-Oxo-4-phenyl}-butyl-1-(3,3-dimethyl-1,2-dioxobutyl)pyrrolidine;
(2*S*)-2-({1-Oxo-4-phenyl}-butyl-1-(2-cyclohexyl-1, 2-dioxoethyl) pyrrolidine;
2-({1-Oxo-4-phenyl}-butyl-1-(3,3-dimethyl-1,2-dioxobutyl)pyrrolidine; and
1-{ (2*S*)-2-[5, 5-di(4-Fluorophenyl)pentanoyl]-2-pyrrolidine}-3,3-dimethyl-1,2-pentanedione;
or a pharmaceutically acceptable salt, ester, or solvate thereof.

8. The use of claim 5, wherein Z is S.

9. The use of claim 8, wherein the compound is selected from the group consisting of:
2-Phenyl-1-ethyl (2*S*)-1-(3,3-dimethyl-1,2-dioxopentyl)-2-pyrrolidinecarbothioate;
(3-Thioindolyl)methyl (2*S*)-1-(3,3-dimethyl-1,2-dioxopentyl)-2-pyrrolidinecarbothioate;
2-Phenyl-1-ethyl (2*S*)-1-(2-cyclohexyl-1,2-dioxoethyl)-2-pyrrolidinecarbothioate;
2-Phenyl-1-ethyl (2*S*)-1-(1-cyclopentyl-1,2-dioxoethyl)-2-pyrrolidinecarbothioate;
3-Phenyl-1-propyl (2*S*)-1-(3,3-dimethyl-1,2-dioxopentyl)-2-pyrrolidinecarbothioate;
3-(3-Pyridyl)-1-propyl (2*S*)-1-(3,3-dimethyl-1,2-dioxoethyl)-2-pyrrolidinecarbothioate;
3-Phenyl-1-propyl (2*S*)-1-(2-cyclohexyl-1,2-dioxoethyl)-2-pyrrolidinecarbothioate;
4-Phenyl-1-butyl (2*S*)-1-(2-cyclohexyl-1,2-dioxoethyl)-2-pyrrolidinecarbothioate;
4-Phenyl-1-butyl (2*S*)-1-(3,3-dimethyl-1,2-dioxopentyl)-2-pyrrolidinecarbothioate;
3-(3-Pyridyl)-1-propyl (2*S*)-1-(2-cyclohexyl-1,2-dioxoethyl)-2-pyrrolidinecarbothioate;
3,3-Diphenyl-1-propyl (2*S*)-1-(3,3-dimethyl-1,2-dioxopentyl)-2-pyrrolidinecarbothioate;
3,3-Diphenyl-1-propyl (2*S*)-1-(2-Cyclohexyl-1,2-dioxoethyl)-2-pyrrolidinecarbothioate;
3-(*para*-Methoxyphenyl)-1-propyl (2*S*)-1-(3,3-diinethyl-1,2-dioxopentyl)-2-pyrrolidinecarbothioate;
3,3-Di(*para*-Fluoro)phenyl-1-propyl (2*S*) -1- (3, 3-dimethyl-1,2-dioxopentyl)-2-pyrrolidinecarbothioate;
4, 4-Di (para-fluorophenyl) butyl 1-(3,3-dimethyl-2-oxopentanoyl)-2-pyrrolidinecarbothioate;
3-(1-Naphthyl)propyl (2*S*)-1-(3,3-dimethyl-2-oxopentanoyl)-2-pyrrolidinecarbothioate;.
2,2-Diphenylethyl (2*S*)-1-(3,3-dimethyl-2-oxopentanoyl)tetrahydro-1H-2-pyrrolidinecarbothioate;
3-[4-(Trifluoromethyl)phenyl]propyl (2*S*)-1-(3,3-dimethyl-2-oxopentanoyl)-2-pyrrolidinecarbothioate;
3-(2-Naphthyl)propyl (2*S*) -1- (3, 3-dimethyl-2-oxopentanoyl)-2-pyrrolidinecarbothioate;
3-(3-Chlorophenyl)propyl (2*S*)-1-(3,3-dimethyl-2-oxopentanoyl)-2-pyrrolidinecarbothioate;
3-[3- (Trifluoromethyl) phenyllpropyl (2*S*) -1- (3, 3-dimethyl-2-oxopentanoyl)-2-pyrrolidinecarbothioate;
3-(1-Biphenyl)propyl (2*S*)-1-(3,3-dimethyl-2-oxopentanoyl)-2-pyrrolidinecarbothioate;
3-(2-Fluorophenyl)propyl (2*S*)-1-(3,3-dimethyl-2-oxopentanoyl)-2-pyrrolidinecarbothioate;
3-(3-Fluorophenyl)propyl (2*S*)-1-(3,3-dimethyl-2-oxopentanoyl)-2-pyrrolidinecarbothioate;
3-(2-Chlorophenyl)propyl (2*S*)-1-(3, 3-dimethyl-2-oxopentanoyl)-2-pyrrolidinecarbothioate; and
3- (3, 4-Dimethoxyphenyl.) propyl (2*S*)-1-(3,3-dimethyl-2-oxopentanoyl)-2-pyrrolidinecarbothioate;
or a pharmaceutically acceptable salt, ester, or solvate thereof.

10. The use of claim 9, wherein the compound is 3,3-Diphenyl-1-propyl (2*S*)-1-(3,3-dimethyl-1,2-dioxopentyl)-2-pyrrolidinecarbothioate or a pharmaceutically acceptable salt, ester, or solvate thereof.

11. The use of claim 9, wherein the compound is 3- (1-Naphthyl) propyl (2*S*)-1-(3,3-dimethyl-2-oxopentanoyl)-2-pyrrolidinecarbothioate or a pharmaceutically acceptable salt, ester, or solvate thereof.

12. The use of claim 9, wherein the compound is 2,2-Diphenylethyl (2*S*)-1-(3,3-dimethyl-2-oxopentanoyl)tetrahydro-1H-2-pyrrolidinecarbothioate or a pharmaceutically acceptable salt, ester, or solvate thereof.

13. The use of claim 9, wherein the compound is 3-[4-(Trifluoromethyl)phenyl]propyl (2S)-1-(3,3-dimethyl-2-oxopentanoyl)-2-pyrrolidinecarbothioate or a pharmaceutically acceptable salt, ester, or solvate thereof.

14. The use of claim 9, wherein the is 3-(2-Naphthyl)propyl (2*S*)-1-(3,3-dimethyl-2-oxopentanoyl)-2-pyrrolidinecarbothioate or a pharmaceutically acceptable salt, ester, or solvate thereof.

15. The use of claim 4, wherein:
n is 1; and
X is S.

16. The use of claim 15, wherein Z is CH₂.

17. The use of claim 15, wherein Z is S.

18. The use of claim 4, wherein:
n is 2; and
X is O.

19. The use of claim 18, wherein Z is CH₂.

20. The use of claim 19, wherein the compound is selected from the group consisting of:
2-({1-Oxo-6-phenyl}-hexyl-1-(2-cyclohexyl-1,2-dioxoethyl)piperidine; and
3,3-Dimethyl-1-[2-(4-phenylpentanoyl)piperidino]-1,2-pentanedione;
or a pharmaceutically acceptable salt, ester, or solvate thereof.

21. The use of claim 18, wherein Z is S.

22. The use of claim 21, wherein the compound is selected from the group consisting of:
2-Phenyl-1-ethyl 1-(3,3-dimethyl-1,2-dioxopentyl)-2-piperidine carbothioate;
2-Phenyl-1-ethyl 1-(2-phenyl-1,2-dioxoethyl)-2-piperidine carbothioate;
3-Phenyl-1-propyl 1-(3,3-dimethyl-1,2-dioxobutyl)-2-piperidine carbothioate;
4-Phenyl-1-butyl 1-(1,2-dioxo-3,3-dimethylbutyl)-2-piperidine ecarbothioate;
1,5-Diphenyl-3-pentyl 1-(3,3-dimethyl-1,2-dioxopentyl)-2-piperidinecarbothioate;
1,5-Diphenyl-3-mercaptopentyl 1-(3-phenyl-1,2-dioxoethyl)-2-piperidinecarbothioate;
3-(para-Methoxyphenyl)-1-propyl 1-(1,2-dioxo-3,3-dimethyl pentyl)piperidine-2-carbothioate;
3-(*para*-Methoxyphenyl)-1-propyl 1- (2-phenyl-1, 2-dioxo ethyl)piperidine-2-carbothioate;
3-(1-Naphthyl)-1-propyl 1-(3,3-dimethyl-1,2-dioxo pentyl)piperidine-2-carbothioate;
2,2-Diphenylethyl (2S)-1-(3,3-dimethyl-2-oxopentanoyl)-2-piperidinecarbothioate;
3,3-Diphenylpropyl 1-(3,3-dimethyl-2-oxopentanoyl)-2-piperidinecarbothioate;
3-(2-Naphthyl)propyl (*2R,S)*-1-(3,3-dimethyl-2-oxopentanoyl)-2-piperidinecarbothioate;
4-Phenylbutyl 1-(3,3-dimethyl-2-oxopentanoyl)-2-piperidine carbothioate;
3-Phenylpropyl 1-(3,3-dimethyl-2-oxopentanoyl)-2-piperidine carbothioate;
3-(2-Chlorophenyl)propyl 1-(3,3-dimethyl-2-oxopentanoyl)-2-piperidinecarbothioate;
3-(2-Fluorophenyl)propyl 1-(3,3-dimethyl-2-oxopentanoyl)-2-piperidinecarbothioate; and
3-(3-Fluorophenyl)propyl 1-(3,3-dimethyl-2-oxopentanoyl)-2-piperidinecarbothioate;
or a pharmaceutically acceptable salt, ester, or solvate thereof.

23. The use of claim 22, wherein the compound is 3,3-Diphenylpropyl 1-(3,3-dimethyl-2-oxopentanoyl)-2-piperidinecarbothioate or a pharmaceutically acceptable salt, ester, or solvate thereof.

24. The use of claim 4, wherein:
n is 2; and
X is S.

25. The use of claim 24, wherein Z is CH₂.

26. The use of claim 24, wherein Z is S.

27. The use of claim 24, wherein Z is CHR₁.

28. The use of claim 27, wherein the compound is 2-({1-Oxo.-[2-{2'-phenyl}ethyl]-4-phenyl}-butyl-1-(3,3-dimethyl-1,2-dioxobutyl.)piperidine, or a pharmaceutically acceptable salt, ester, or solvate thereof.

29. The use of claim 1, wherein the compound is of formula III 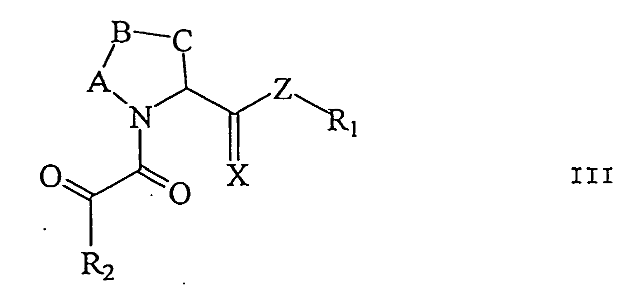 or a pharmaceutically acceptable salt, ester, or solvate thereof, wherein:
A, B, and C are independently CH₂, O, S, SO, SO₂, NH or NR₂;
X is O or S;
Z is S, CH₂, CHR₁, or CR₁R₃;
R₁ and R₃ are independently C₁-C₆ straight or branched chain alkyl or C₂-C₆ straight or branched chain alkenyl,
wherein said alkyl or alkenyl is substituted with one or more substituent (s) independently selected from the group consisting of (Ar₁)ₙ, C₁-C₆ straight or branched chain alkyl or C₂₋C₆ straight or branched chain alkenyl substituted with (Ar₁)ₙ, C₃-C₈ cycloalkyl, C₁₋C₆ straight or branched chain alkyl or C₂-C₆ straight or branched chain alkenyl substituted with C₃-C₈ cycloalkyl, and Ar₂;
R₂ is either C₁-C₉ straight or branched chain alkyl or C₂-C₉ straight or branched chain alkenyl, C₃-C₈ cycloalkyl, C₅-C₇ cycloalkenyl or Art,
wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring,
wherein said ring is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino,
wherein the individual ring size is 5-8 members,
and wherein the heterocyclic ring contains 1-6 heteroatom(s) independently selected from the group consisting of O, N, and S.

30. The use of claim 29, wherein:
A is CH₂;
B is CH₂ or S;
C is CH₂ or NH;
X is O or S;
Z is S or CH₂;
R₁ is 2-phenethyl or 3-phenylpropyl; and
R₂ is 1,1-dimethylpropyl.

31. The use of claim 1, wherein the compound is of formula IV 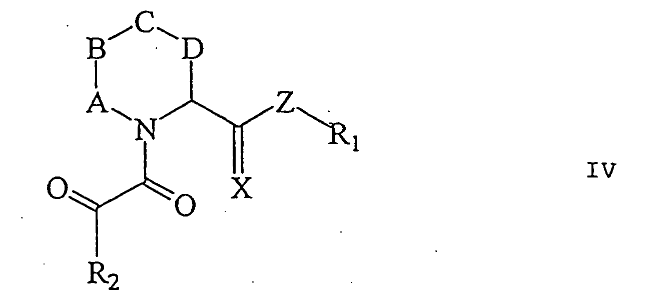 or a pharmaceutically acceptable salt, ester, or solvate thereof, wherein:
A, B, C and D are independently CH₂, 0, S, SO, SO₂, NH, or NR₂;
X is O or S;
Z is S, CH₂, CHR₁, or CR₁R₃;
R₁ and R₃ are independently C₁-C₆ straight or branched chain alkyl or C₂-C₆ straight or branched chain alkenyl,
wherein said alkyl or alkenyl is substituted with one or more substituent(s) independently selected from the group consisting of (Ar₁)ₙ, C₁-C₆ straight or branched chain alkyl or C₂₋C₆ straight or branched chain alkenyl substituted with (Ar₁)ₙ, C₃-C₈ cycloalkyl, C₁₋C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl or C₂-C₉ straight or branched chain alkenyl, C₃-C₈ cycloalkyl, C₅-C₇ cycloalkenyl or Ar₁,
wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring,
wherein said ring is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino,
wherein the individual ring size is 5-8 members,
and wherein the heterocyclic ring contains 1-6 heteroatom(s) independently selected from the group consisting of O, N, and S.

32. The use of claim 31, wherein:
A is CH₂;
B is CH₂;
C is S or O;
D is CH₂;
X is O;
Z is CH₂ or S;
R₁ is 3-phenylpropyl or 2-phenethyl; and
R₂ is 1,1-dimethylpropyl.

33. The use of any one of claims 1-32, wherein the compound is non-immunosuppressive.

34. The use of any one of claims 1-32, wherein the compound has an affinity for an FKBP-type immunophilin .

35. The use of claim 34, wherein
the FKBP-type immunophilin is FKBP-12.

36. The use of any one of claims 1-32, wherein the compound is immunosuppressive.

## Patentansprüche

1. Verwendung einer Verbindung zur Herstellung einer pharmazeutischen Zusammensetzung oder eines Medikaments zur Behandlung von Alopecia oder zur Förderung des Haarwachstums bei einem Tier, das dessen bedarf, wobei diese Verbindung die Formel (V) hat: 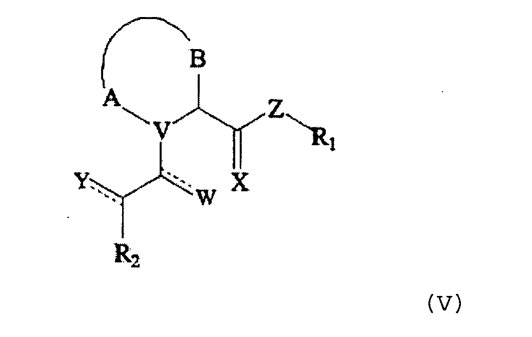 oder eines pharmazeutisch akzeptablen Salzes, Esters oder Solvats davon, worin gilt:
V ist C, N, oder S;
A und B, zusammengenommen mit V und das Kohlenstoffatom, an das sie jeweils gebunden sind, bilden einen 5-7 gliedrigen gesättigten oder ungesättigten heterozyklischen Ring der ein oder mehrere Heteroatom(e) enthält, welche unabhängig ausgewählt werden aus der Gruppe bestehend aus O, S, SO, SO₂, N, NH, und NR₄;
R₄ ist entweder C₁-C₉ lineares oder verzweigtes Alkyl, C₂-C₉ lineares oder verzweigtes Alkenyl, C₃-C₉ Cycloalkyl, C₅-C₇ Cycloalkenyl, oder Ar₃,
wobei R₄ entweder unsubstituiert oder mit einem oder mehreren Substituenten substituiert ist, welche unabhängig ausgewählt werden aus der Gruppe bestehend aus Halogen, Halogenalkyl, Carbonyl, Carboxy, Hydroxy, Nitro, Trifluormethyl, C₁-C₆ lineares oder verzweigtes Alkyl, C₂-C₆ lineares oder verzweigtes Alkenyl, C₁-C₄ Alkoxy, C₂-C₄ Alkenyloxy, Phenoxy, Benzyloxy, Thioalkyl, Alkylthio, Sulfhydryl, Amino, Alkylamino, Aminoalkyl, Aminocarboxyl, und Ar₄;
Ar₃ und Ar₄ sind unabhängig ein alicyclischer oder aromatischer, mono-, bi- oder tricyclischer, carbo- oder heterozyklischer Ring; wobei die jeweilige Ringgröße 5-8 Ringglieder beträgt; und wobei der heterozyklischer Ring 1-6 Heteroatome enthält, die unabhängig aus der Gruppe bestehend aus O, N, und S ausgewählt werden;
X ist entweder O oder S
Z ist entweder S, CH₂, CHR₁ oder CR₁R_{3;}
W und Y sind unabhängig O, S, CH₂ oder H₂;
R₁ und R₃ sind unabhängig C₁-C₆ lineares öder verzweigtes Alkyl oder C₂-C₆ lineares oder verzweigtes Alkenyl,
wobei dieses Alkyl oder Alkenyl mit einem oder mehreren Substituenten substituiert ist, die unabhängig ausgewählt werden aus der Gruppe bestehend aus (Ar₁)ₙ,C₁-C₆ linearem oder verzweigtem Alkyl oder C₂-C₆ linearem oder verzweigtem Alkenyl substituiert mit (Ar₁)ₙ, C₃-C₈ Cycloalkyl, C₁-C₆ linearem oder verzweigtem Alkyl oder C₂-C₆ linearem oder verzweigtem Alkenyl substituiert mit C₃-C₈ Cycloalkyl, und Ar₂;
n ist 1 oder 2;
R₂ ist entweder C₁-C₉ lineares oder verzweigtes Alkyl, C₂-C₉ lineares oder verzweigtes Alkenyl, C₃-C₈ Cycloalkyl, C₅-C₇ Cycloalkenyl oder Ar₁,
wobei dieses Alkyl, Alkenyl, Cycloalkyl oder Cycloalkenyl entweder unsubstituiert oder mit einem oder mehreren Substituenten substituiert ist, welche unabhängig ausgewählt werden aus der Gruppe bestehend aus C₁-C₄ linearem oder verzweigtem Alkyl, C₂-C₄ linearem oder verzweigtem Alkenyl, und Hydroxy; und
Ar₁ und Ar₂ sind unabhängig ein alicyclischer oder aromatischer, mono-, bi- oder tricyclischer, carbo- oder heterozyklischer Ring,
wobei dieser Ring entweder unsubstituiert oder mit einem oder mehreren Substituenten substituiert ist, welche unabhängig ausgewählt werden aus der Gruppe bestehend aus Halogen, Hydroxy, Nitro, Trifluormethyl, C₁-C₆ linearem oder verzweigtem Alkyl, C₂-C₆ linearem oder verzweigtem Alkenyl, C₁-C₄ Alkoxy, C₂-C₄ Alkenyloxy, Phenoxy, Benzyloxy, und Amino;
wobei die jeweilige Ringgröße 5-8 Ringglieder beträgt;
und wobei der heterozyklische Ring 1-6 Heteroatome enthält, unabhängig ausgewählt aus der Gruppe bestehend aus O, N, und S.

2. Verwendung gemäß Anspruch 1, wobei die Verbindung die Formel (I) hat 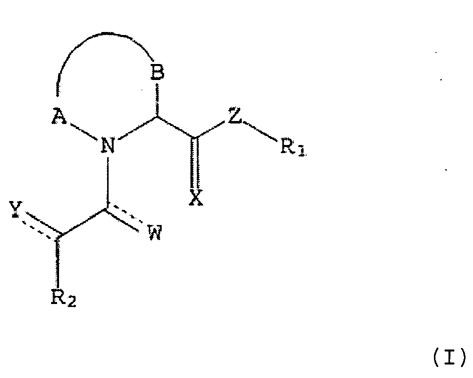 oder eines pharmazeutisch akzeptablen Salzes, Esters oder Solvats davon, worin gilt:
A und B, zusammengenommen mit dem Stickstoffatom und dem Kohlenstoffatom, an das sie jeweils gebunden sind, bilden einen 5-7 gliedrigen gesättigten oder ungesättigten heterozyklischen Ring, der ein oder mehrere weitere O, S, SO, SO₂, N, NH, oder NR₂ Heteroatom(e) enthält;
X ist entweder O oder S;
Z ist entweder S, CH₂, CHR₁ oder CR₁R₃;
W und Y sind unabhängig O, S, CH₂ oder H₂;
R₁ und R₃ sind unabhängig C₁-C₆ lineares oder verzweigtes Alkyl oder C₂-C₆ lineares oder verzweigtes Alkenyl,
wobei dieses Alkyl oder Alkenyl mit einem oder mehreren Substituenten substituiert ist, die unabhängig ausgewählt werden aus der Gruppe bestehend aus (Ar₁)ₙ, C₁-C₆ linearem oder verzweigtem Alkyl oder C₂-C₆ linearem oder verzweigtem Alkenyl substituiert mit (Ar₁)ₙ, C₃-C₈ Cycloalkyl, C₁-C₆ linearem oder verzweigtem Alkyl oder C₂-C₆ linearem oder verzweigtem Alkenyl substituiert mit C₃-C₈ Cycloalkyl, und Ar₂;
n ist 1 oder 2;
R₂ ist entweder C₁-C₉ lineares oder verzweigtes Alkyl,
C₂-C₉ lineares oder verzweigtes Alkenyl, C₃-C₈ Cycloalkyl, C₅-C₇ Cycloalkenyl oder Ar₁,
wobei diese Alkyl-, Alkenyl-, Cycloalkyl- oder Cycloalkenylgruppen entweder unsubstituiert oder mit einem oder mehreren Substituenten substituiert sind, welche unabhängig ausgewählt werden aus der Gruppe bestehend aus C₁-C₄ linearem oder verzweigtem Alkyl, C₂-C₄ linearem oder verzweigtem Alkenyl, und Hydroxy;
und Ar₁ und Ar₂ sind unabhängig ein alicyclischer oder aromatischer, mono-, bi- oder tricyclischer, carbo- oder heterozyklischer Ring,
wobei dieser Ring entweder unsubstituiert oder mit einem oder mehreren Substituenten substituiert ist, welche unabhängig ausgewählt werden aus der Gruppe bestehend aus Halogen, Hydroxy, Nitro, Trifluormethyl, C₁-C₆ linearem oder verzweigtem Alkyl, C₂-C₆ linearem oder verzweigtem Alkenyl, C₁-C₄ Alkoxy, C₂-C₄ Alkenyloxy, Phenoxy, Benzyloxy, und Amino;
wobei die jeweilige Ringgröße 5-8 Ringglieder beträgt;
und wobei der heterozyklische Ring 1-6 Heteroatome enthält, die unabhängig aus der Gruppe bestehend aus O, N, und S ausgewählt werden.

3. Verwendung gemäß Anspruch 2, worin der mono- oder bicyclische, carbo- oder heterozyklische Ring ausgewählt ist aus der Gruppe bestehend aus Naphthyl, Indolyl, Furyl, Thiazolyl, Thienyl, Pyridyl, Chinolinyl, Isochinolinyl, Fluorenyl, und Phenyl.

4. Verwendung gemäß Anspruch 1, worin die Verbindung die Formel (II) hat: 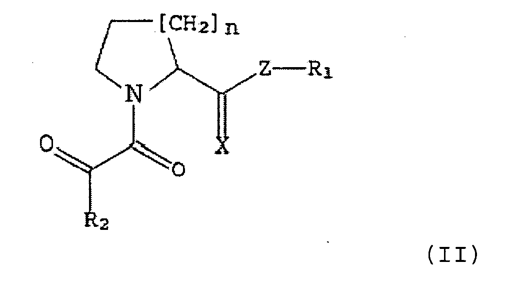 oder eines pharmazeutisch akzeptablen Salzes, Esters oder Solvats davon, worin gilt:
n ist 1 oder 2;
X ist O oder S;
Z ist ausgewählt aus der Gruppe bestehend aus S, CH₂, CHR₁, und CR₁R₃;
R₁ und R₃ sind unabhängig ausgewählt aus der Gruppe bestehend aus C₁-C₅ linearem oder verzweigtem Alkyl, C₂-C₅ linearem oder verzweigtem Alkenyl, und Ar₁,
wobei R₁ unsubstituiert oder mit einem oder mehreren Substituenten substituiert ist, unabhängig ausgewählt aus der Gruppe, bestehend aus Halogen, Nitro, C₁-C₆ linearem oder verzweigtem Alkyl, C₂-C₆ linearem oder verzweigtem Alkenyl, Hydroxy, C₁-C₄ Alkoxy, C₂-C₄ Alkenyloxy, Phenoxy, Benzyloxy, Amino, und Ar₁;
R₂ ist ausgewählt aus der Gruppe bestehend aus C₁-C₉ linearem oder verzweigtem Alkyl, C₂-C₉ linearem oder verzweigtem Alkenyl, C₃-C₈ Cycloalkyl, C₅-C₇ Cycloalkenyl, und Ar₁; und
Ar₁ ist Phenyl, Benzyl, Pyridyl, Fluorenyl, Thioindolyl oder Naphthyl,
wobei Ar₁ unsubstituiert oder mit einem oder mehreren Substituenten substituiert ist, welche unabhängig ausgewählt werden aus der Gruppe bestehend aus Halogen, Trifluormethyl, Hydroxy, Nitro, C₁-C₆ linearem oder verzweigtem Alkyl, C₂-C₆ linearem oder verzweigtem Alkenyl, C₁-C₄ Alkoxy, C₂-C₄ Alkenyloxy, Phenoxy, Benzyloxy, und Amino.

5. Verwendung gemäß Anspruch 4, worin gilt:
n ist 1 und X ist O.

6. Verwendung gemäß Anspruch 5, worin Z CH₂ ist,

7. Verwendung gemäß Anspruch 6, worin die Verbindung ausgewählt ist aus der Gruppe bestehend aus:
(2S)-2-({1-Oxo-5-phenyl}-pentyl-1-(3,3-dimethyl-1,2-dioxopentyl)pyrrolidin;
3,3-Dimethyl-1-[(2S)-2-(5-(3-pyridyl)pentanoyl)-1-pyrrolidin]-1,2-pentandion;
(25) -2- ({1-Oxo-4-phenyl)-1-butyl-1- (3,3-dimethyl-1,2-dioxobutyl)pyrrolidin;
(2S)-2-({1-Oxo-4-phenyl}-butyl-1-(2-cyclohexyl-1,2-dioxoethyl)pyrrolidin;
2-({1-Oxo-4-phenyl}-butyl-1-(3,3-dimethyl-1,2-dioxobutyl)pyrrolidin;
1-{(2S)-2-[5,5-di(4-Fluorophenyl)pentanoyl]-2-pyrrolidin}-3,3-dimethyl-1,2-pentandion;
und pharmazeutisch annehmbaren Salzen, Estern und Solvaten davon.

8. Verwendung gemäß Anspruch 5, worin Z S ist.

9. Verwendung gemäß Anspruch 8, worin die Verbindung ausgewählt ist aus der Gruppe bestehend aus:
2-Phenyl-1-ethyl-(2S)-1-(3,3-dimethyl-1,2-dioxopentyl)-2-pyrrolidincarbothioat;
(3-Thioindolyl)methyl-(2S)-1-(3,3-dimethyl-1,2-dioxopentyl)-2-pyrrolidincarbothioat;
2-Phenyl-1-ethyl-(2S)-1-(2-cyclohexyl-1,2-dioxoethyl)-2-pyrrolidincarbothioat;
2-Phenyl-1-ethyl-(2S)-1-(1-cyclopentyl-1,2-dioxoethyl)-2-pyrrolidincarbothioat;
3-Phenyl-1-propyl-(2S)-1-(3,3-dimethyl-1,2-di-oxopentyl)-2-pyrrolidincarbothioat;
3-(3-Pyridyl)-1-propyl-(2S)-1-(3,3-dimethyl-1,2-dioxopentyl)-2-pyrrolidincarbothioat;
3-Phenyl-1-propyl-(2S)-1-(2-cyclohexyl-1,2-dioxoethyl)-2-pyrrolidincarbothioat;
4-Phenyl-1-butyl-(2S)-1-(2-cyclohexyl-1,2-dioxoethyl)-2-pyrrolidincarbothioat;
4-Phenyl-1-butyl-(2S)-1-(3,3-dimethyl-1,2-dioxopentyl)-2-pyrrolidincarbothioat;
3-(3-Pyridyl)-1-propyl-(2S)-1-(2-cyclohexyl-1,2-dioxoethyl)-2-pyrrolidincarbothioat;
3,3-Diphenyl-1-propyl-(2S)-1-(3,3-dimethyl-1,2-dioxopentyl)-2-pyrrolidincarbothioat;
3,3-Diphenyl-1-propyl-(2S)-1-(2-Cyclohexyl-1,2-dioxoethyl)-2-pyrrolidincarbothioat;
3-(para-Methoxyphenyl)-1-propyl-(2S)-1-(3,3-dimethyl-1,2-dioxopentyl)-2-pyrrolidincarbothioat;
3,3-Di(para-fluor)phenyl-1-propyl-(2S)-1-(3,3-dimethyl-1,2-dioxopentyl)-2-pyrrolidincarbothioat;
4,4-Di(para-fluorphenyl)butyl-1-(3,3-dimethyl-2-oxo-pentanoyl)-2-pyrrolidincarbothioat;
3-(1-Naphthyl)propyl-(2S)-1-(3,3-dimethyl-2-oxopentanoyl)-2-pyrrolidincarbothioat;
2,2-Diphenylethyl-(2S)-1-(3,3-dimethyl-2-oxopentanoyl)-tetrahydro-1H-2-pyrrolidincarbothioat;
3-[4-(Trifluormethyl)phenyl]propyl-(2S)-1-(3,3-dimethyl-2-oxopentanoyl)-2-pyrrolidincarbothioat;
3-(2-Naphthyl)propyl-(2S)-1-(3,3-dimethyl-2-oxopentanoyl)-2-pyrrolidincarbothioat;
3-(3-Chlorphenyl)propyl-(2S)-1-(3,3-dimethyl-2-oxopentanoyl)-2-pyrrolidincarbothioat;
3-[3-(Trifluormethyl)phenyl]propyl-(2S)-1-(3,3-dimethyl-2-oxopentanoyl)-2-pyrrolidincarbothioat;
3-(1-Biphenyl)propyl-(2S)-1-(3,3-dimethyl-2-oxopentanoyl)-2-pyrrolidincarbothioat;
3-(2-Fluorphenyl)propyl-(2S)-1-(3,3-dimethyl-2-oxopentanoyl)-2-pyrrolidincarbothioat;
3-(3-Fluorphenyl)propyl-(2S)-1-(3,3-dimethyl-2-oxopentanoyl)-2-pyrrolidincarbothioat;
3-(2-Chlorphenyl)propyl-(2S)-1-(3,3-dimethyl-2-oxopentanoyl)-2-pyrrolidincarbothioat;
3-(3,4-Dimethoxyphenyl)propyl-(2S)-1-(3,3-dimethyl-2-oxopentanoyl)-2-pyrrolidincarbothioat;
und pharmazeutisch annehmbaren Salzen, Estern und Solvaten davon.

10. Verwendung gemäß Anspruch 9, worin die Verbindung 3,3-Diphenyl-1-propyl-(2S)-1-(3,3-dimethyl-1,2-dioxopentyl)-2-pyrrolidincarbothioat oder ein pharmazeutisch akzeptables Salz, Ester oder Solvat davon ist.

11. Verwendung gemäß Anspruch 9, worin die Verbindung 3-(1-Naphthyl)propyl-(2*S*)-1-(3,3-dimethyl-2-oxopentanoyl)-2 pyrrolidincarbothioat oder ein pharmazeutisch akzeptables Salz, Ester oder Solvat davon ist.

12. Verwendung gemäß Anspruch 9, worin die Verbindung 2,2-Diphenylethyl-(2S)-1-(3,3-dimethyl-2-oxopentanoyl)tetrahydro-1H-2-pyrrolidincarbothioat oder ein pharmazeutisch akzeptables Salz, Ester oder Solvat davon ist.

13. Verwendung gemäß Anspruch 9, worin die Verbindung 3-[4-(Trifluormethyl)phenyl]propyl-(2S)-1-(3,3-dimethyl-2-oxopentanoyl)-2-pyrrolidincarbothioat oder ein pharmazeutisch akzeptables Salz, Ester oder Solvat davon ist.

14. Verwendung gemäß Anspruch 9, worin die Verbindung 3-(2-Naphthyl)propyl-(2S)-1-(3,3-dimethyl-2-oxopentanoyl-2-pyrrolidincarbothioat oder ein pharmazeutisch akzeptables Salz, Ester oder Solvat davon ist.

15. Verwendung gemäß Anspruch 4, worin: n = 1; und X S ist.

16. Verwendung gemäß Anspruch 15, worin Z CH₂ ist.

17. Verwendung gemäß Anspruch 15, worin Z S ist.

18. Verwendung gemäß Anspruch 4, worin n = 2; und X O ist.

19. Verwendung gemäß Anspruch 18, worin Z CH₂ ist.

20. Verwendung gemäß Anspruch 19, worin die Verbindung ausgewählt ist aus der Gruppe bestehend aus:
2-({1-Oxo-6-phenyl}-hexyl-1-(2-cyclohexyl-1,2-dioxoethyl)piperidin;
3,3-Dimethyl-1-[2-(4-phenylpentanoyl)piperidino]-1,2-pentandion;
und pharmazeutisch annehmbaren Salzen, Estern und Solvaten davon.

21. Verwendung gemäß Anspruch 18, worin Z S ist.

22. Verwendung gemäß Anspruch 21, worin die Verbindung ausgewählt ist aus der Gruppe bestehend aus:
2-Phenyl-1-ethyl 1-(3,3-dimethyl-1,2-dioxopentyl)-2-piperidincarbothioat;
2-Phenyl-1-ethyl 1-(2-phenyl-1,2-dioxoethyl)-2-piperidincarbothioat;
3-Phenyl-1-propyl 1-(3,3-dimethyl-1,2-dioxobutyl)-2-piperidincarbothioat;
4-Phenyl-1-butyl 1-(1,2-dioxo-3,3-dimethylbutyl)-2-piperidincarbothioat;
1,5-Diphenyl-3-pentyl 1-(3,3-dimethyl-1,2-dioxopentyl)-2-piperidincarbothioat;
1,5-Diphenyl-3-mercaptopentyl 1-(3-phenyl-1,2-dioxoethyl)-2-piperidincarbothioat;
3-(para-Methoxyphenyl)-1-propyl 1-(1,2-dioxo-3,3-dimethylpentyl)piperidin-2-carbothioat;
3-(para-Methoxyphenyl)-1-propyl 1-(2-phenyl-1,2-dioxoethyl)piperidin-2-carbothiöat;
3-(1-Naphthyl)-1-propyl 1-(3,3-dimethyl-1,2-dioxopentyl)-piperidin-2-carbothioat;
2,2-Diphenylethyl-(2S)-1-(3,3-dimethyl-2-oxopentanoyl)-2-piperidincarbothioat;
3,3-Diphenylpropyl-1-(3,3-dimethyl-2-oxopentanoyl)-2-piperidincarbothioat;
3-(2-Naphthyl)propyl-(2R,S)-1-(3,3-dimethyl-2-oxopentanoyl)-2-piperidincarbothioat;
4-Phenylbutyl-1-(3,3-dimethyl-2-oxopentanoyl)-2-piperidincarbothioat;
3-Phenylpropyl-1-(3,3-dimethyl-2-oxopentanoyl)-2-piperidincarbothioat;
3-(2-Chlorphenyl)propyl-1-(3,3-dimethyl-2-oxopentanoyl)-2-piperidincarbothioat;
3-(2-Fluorphenyl)propyl-1-(3,3-dimethyl-2-oxopentanoyl)-2-piperidincarbothioat; und
3-(3-Fluorphenyl)propyl-1-(3,3-dimethyl-2-oxopentanoyl)-2-piperidincarbothioat;
oder pharmazeutisch annehmbaren Salzen, Estern und Solvaten davon.

23. Verwendung gemäß Anspruch 22, worin die Verbindung 3,3-Diphenylpropyl 1-(3,3-dimethyl-2-oxopentanoyl)-2-piperidincarbothioat oder ein pharmazeutisch akzeptables Salz, Ester oder Solvat davon ist.

24. Verwendung gemäß Anspruch 4, worin: n = 2; und X S ist.

25. Verwendung gemäß Anspruch 24, worin Z CH₂ ist.

26. Verwendung gemäß Anspruch 24, worin Z S ist.

27. Verwendung gemäß Anspruch 24, worin Z CHR₁ ist.

28. Verwendung gemäß Anspruch 27, worin die Verbindung 2-({1-Oxo-[2-{2'-phenyl}ethyl]-4-phenyl}-butyl-1-(3,3-dimethyl-1,2-dioxobutyl)piperidin oder ein pharmazeutisch akzeptables Salz, Ester oder Solvat davon ist.

29. Verwendung gemäß Anspruch 1, wobei die Verbindung die Formel (III) hat: 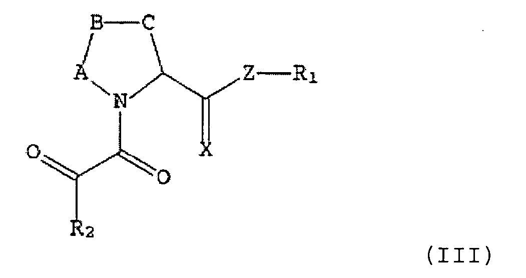 oder eines pharmazeutisch akzeptablen Salzes, Esters oder Solvats davon, worin gilt:
A, B, und C sind unabhängig CH₂, O, S, SO, SO₂, NH oder NR₂;
X ist O oder S;
Z ist S, CH₂, CHR₁, oder CR₁R₃;
R₁ und R₃ sind unabhängig C₁-C₆ lineares oder verzweigtes Alkyl oder C₂-C₆ lineares oder verzweigtes Alkenyl,
wobei das Alkyl oder Alkenyl mit einem oder mehreren Substituenten substituiert ist, die unabhängig ausgewählt werden aus der Gruppe bestehend aus (Ar₁)ₙ, C₁-C₆ linearem oder verzweigtem Alkyl oder C₂₋C₆ linearem oder verzweigtem Alkenyl substituiert mit (Ar₁)ₙ, C₃-C₈ Cycloalkyl, C₁-C₆ linearem oder verzweigtem Alkyl oder C₂-C₆ linearem oder verzweigtem Alkenyl substituiert mit C₃-C₈ Cycloalkyl, und Ar₂;
R₂ ist entweder C₁-C₉ lineares oder verzweigtes Alkyl oder C₂-C₉ lineares oder verzweigtes Alkenyl, C₃-C₈ Cycloalkyl, C₅-C₇ Cycloalkenyl oder Ar₁,
wobei die Alkyl-, Alkenyl-, Cycloalkyl- oder Cycloalkenylgruppen entweder unsubstituiert oder mit einem oder mehreren Substituenten substituiert sind, die unabhängig ausgewählt werden aus der Gruppe bestehend aus C₁-C₄ linearem oder verzweigtem Alkyl, C₂-C₄ linearem oder verzweigtem Alkenyl, und Hydroxy; und Ar₁ und Ar₂ sind unabhängig ein alicyclischer oder aromatischer, mono-, bi- oder tricyclischer, carbo- oder heterozyklischer Ring, wobei dieser Ring entweder unsubstituiert oder mit einem oder mehreren Substituenten substituiert ist, welche unabhängig ausgewählt werden aus der Gruppe bestehend aus Halogen, Hydroxy, Nitro, Trifluormethyl, C₁-C₆ linearem oder verzweigtem Alkyl, C₂-C₆ linearem oder verzweigtem Alkenyl, C₁-C₄ Alkoxy, C₂-C₄ Alkenyloxy, Phenoxy, Benzyloxy, und Amino; wobei die jeweilige Ringgröße 5-8 Ringglieder beträgt; und wobei der heterozyklische Ring 1-6 Heteroatome enthält, die unabhängig aus der Gruppe bestehend aus O, N, und S ausgewählt werden.

30. Verwendung gemäß Anspruch 29, worin gilt:
A ist CH₂;
B ist CH₂ oder S;
C ist CH₂ oder NH;
X ist O oder S;
Z ist S oder CH₂;
R₁ ist 2-Phenethyl oder 3-Phenylpropyl; und
R₂ ist 1,1-Dimethylpropyl.

31. Verwendung gemäß Anspruch 1, wobei die Verbindung die Formel (IV) hat 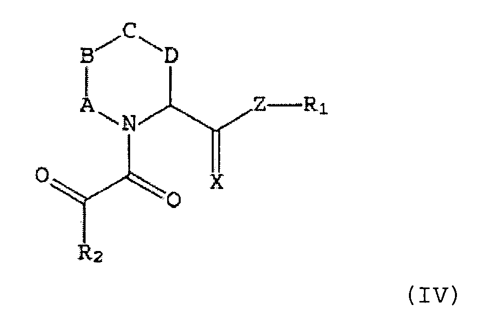 oder eines pharmazeutisch akzeptablen Salzes, Esters oder Solvats davon, worin gilt:
A, B, C und D sind unabhängig CH₂, O, S, SO, SO₂, NH, oder NR₂;
X ist O oder S;
Z ist S, CH₂, CHR₁, oder CR₁R₃;
R₁ und R₃ sind unabhängig C₁-C₆ lineares oder verzweigtes Alkyl oder C₂-C₆ lineares oder verzweigtes Alkenyl,
wobei das Alkyl oder Alkenyl mit einem oder mehreren Substituenten substituiert ist, welche unabhängig ausgewählt werden aus der Gruppe bestehend aus (Ar₁)ₙ, C₁-C₆ linearem oder verzweigtem Alkyl oder C₂-C₆ linearem oder verzweigtem Alkenyl substituiert mit (Ar₁)ₙ, C₃-C₈ Cycloalkyl, C₁-C₆ linearem oder verzweigtem Alkyl oder C₂-C₆ linearem oder verzweigtem Alkenyl substituiert mit C₃-C₈ Cycloalkyl, und Ar₂;
n ist 1 oder 2;
R₂ ist entweder C₁-C₉ lineares oder verzweigtes Alkyl oder C₂-C₉ lineares oder verzweigtes Alkenyl, C₃-C₈ Cycloalkyl, C₅-C₇ Cycloalkenyl oder Ar₁,
wobei die Alkyl-, Alkenyl-, Cycloalkyl- oder Cycloalkenylgruppen entweder unsubstituiert oder mit einem oder mehreren Substituenten substituiert sind, die unabhängig ausgewählt werden aus der Gruppe bestehend aus C₁-C₄ linearem oder verzweigtem Alkyl, C₂-C₄ linearem oder verzweigtem Alkenyl, und Hydroxy; und
Ar₁ und Ar₂ sind unabhängig ein alicyclischer oder aromatischer, mono-, bi- oder tricyclischer, carbo- oder heterozyklischer Ring,
wobei dieser Ring entweder unsubstituiert oder mit einem oder mehreren Substituenten substituiert ist, welche unabhängig ausgewählt werden aus der Gruppe bestehend aus Halogen, Hydroxy, Nitro, Trifluormethyl, C₁-C₆ lineares oder verzweigtes Alkyl, C₂-C₆ lineares oder verzweigtes Alkenyl, C₁-C₄ Alkoxy, C₂-C₄ Alkenyloxy, Phenoxy, Benzyloxy, und Amino; wobei die jeweilige Ringgröße 5-8 Ringglieder beträgt; und wobei der heterozyklische Ring 1-6 Heteroatom(e) enthält, die unabhängig ausgewählt werden aus der Gruppe bestehend aus O, N, und S.

32. Verwendung gemäß Anspruch 31, worin gilt:
A ist CH₂;
B ist CH₂;
C ist S oder O;
D ist CH₂;
X ist O;
Z ist CH₂ oder S;
R₁ ist 3-Phenylpropyl oder 2-Phenethyl; und
R₂ ist 1,1-Dimethylpropyl.

33. Verwendung gemäß einem der Ansprüche 1-32, wobei die Verbindung nicht-immunosuppressiv ist.

34. Verwendung gemäß einem der Ansprüche 1-32, wobei die Verbindung eine Affinität für ein Immunophilin vom FKBP-Typ hat.

35. Verwendung gemäß Anspruch 34, worin das Immunophilin vom FKBP-Typ FKBP-12 ist.

36. Verwendung gemäß einem der Ansprüche 1-32, wobei die Verbindung immunosuppressiv ist.

## Revendications

1. Utilisation d'un composé pour la préparation d'une composition pharmaceuticlue ou d'un médicament pour traiter une alopécie ou favoriser la croissance des cheveux chez un animal en ayant besoin, dans laquelle ledit composé est de la formule V 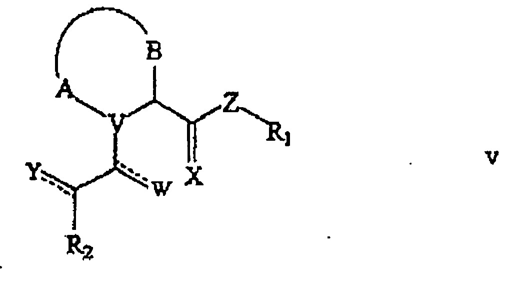 ou un sel, ester ou produit de solvatation pharmaceutiquement acceptable de celle-ci,
dans laquelle :
V est C, N, ou S;
A et B, pris ensemble avec V et l'atome de carbone auxquels ils sont respectivement liés, forment un noyau hétérocyclique saturé ou insaturé de 5 à 7 membres, contenant un ou plusieurs hétéroatomes indépendamment choisis dans le groupe constitué par O, S, SO, SO₂, N, NH, et NR₄;
R₄ est l'un quelconque de un alkyle en C₁-C₉ à chaîne linéaire ou ramifiée, un alcényle en C₂-C₉ à chaîne linéaire ou ramifiée, un cycloalkyle en C₃-C₉, un cycloalcényle en C₅-C₇, ou Ar₃,
où R₄ est soit non substitué, soit substitué avec un ou plusieurs substituants indépendamment choisis dans le groupe constitué par un halogène, un haloalkyle, un carbonyle, un carboxy, un hydroxy, un nitro, un trifluorométhyle, un alkyle en C₁-C₆ à chaîne linéaire ou ramifiée, un alcényle en C₂-C₆ a chaîne linéaire ou ramifiée, un alcoxy en C₁-C₄, un alcényloxy en C₂-C₄, un phénoxy, un benzyloxy, un thioalkyle, un alkylthio, un sulfhydryle, un amino, un alkylamino, un aminoalkyle, un aminocarboxyle, et Ar₄;
Ar₃ et Ar₄ sont indépendamment un noyau carbo- ou hétérocyclique, mono-, bi- ou tricyclique, alicyclique ou aromatique,
où la taille des cycles individuels est de 5 - 8 membres,
et où ledit noyau hétérocyclique contient 1 - 6 hétéroatomes indépendamment choisis dans le groupe constitué par O, N, et S;
X est l'un quelconque de O ou S;
Z est l'un quelconque de S, CH₂, CHR₁ ou CR₁R₃;
W et Y sont indépendamment O, S, CH₂ or H₂;
R₁ et R₃ sont indépendamment un alkyle en C₁-C₆ à chaîne linéaire ou ramifiée ou un alcényle en C₂-C₆ à chaîne linéaire ou ramifiée,
où ledit alkyle ou alcényle est substitué avec un ou plusieurs substituants indépendamamment choisis dans le groupe constitué par (Ar₁)ₙ, un alkyle en C₁-C₆ à chaîne linéaire ou ramifiée ou alcényle en C₂-C₆ à chaîne linéaire ou ramifiée substitué avec (Ar₁)ₙ, un cycloalkyle en C₃-C₈, un alkyle en C₁-C₆ à chaîne linéaire ou ramifiée ou alcényle en C₂-C₆ à chaîne linéaire ou ramifiée substitué avec un cycloalkyle en C₃-C₈, et Ar₂;
n est 1 ou 2;
R₂ est l'un quelconque de un alkyle en C₁-C₉ à chaîne linéaire ou ramifiée, un alcényle en C₂-C₉ à chaîne linéaire ou ramifiée, un cycloalkyle en C₃-C₈, un cycloalcényle en C₅-C₇ ou Ar₁,
où ledit alkyle, alcényle, cycloalkyle ou cycloalcényle est soit non substitué, soit substitué avec un ou plusieurs substituants indépendamment choisis dans le groupe constitué par un alkyle en C₁-C₄ à chaîne linéaire ou ramifiée, un alcényle en C₂-C₄ à chaîne linéaire ou ramifiée et un hydroxy; et
Ar₁ et Ar₂ sont indépendamment un noyau carbo- ou hétérocyclique, mono-, bi- ou tricyclique, alicyclique ou aromatique,
où ledit noyau est soit non substitué, soit substitué avec un ou plusieurs substituants indépendamment choisis dans le groupe constitué par un halogène, un hydroxy, un nitro, un trifluorométhyle, un alkyle en C₁-C₆ à chaîne linéaire ou ramifiée, un alcényle en C₂-C₆ à chaîne linéaire ou ramifiée, un alcoxy en C₁-C₄, un alcényloxy en C₂-C₄, un phénoxy, un benzyloxy, et un amino,
où la taille des cycles individuels est de 5 - 8 membres,
et où le noyau hétérocyclique a 1 à 6 hétéroatomes indépendamment choisis dans le groupe constitué par O**,** N, et S.

2. Utilisation de la revendication 1, dans laquelle le composé est de la formule I 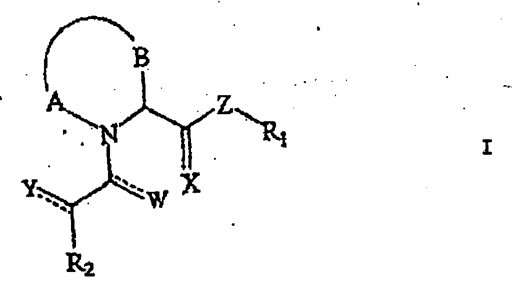 ou un sel, ester ou produit de solvatation pharmaceutiquement acceptable de celle-ci, dans laquelle :
A et B, pris ensemble avec l'azote et l'atome de carbone auxquels ils sont respectivement liés, forment un noyau hétérocyclique saturé ou insaturé de 5 à 7 membres contenant un ou plusieurs hétéroatomes additionnels O, S, SO, SO₂, N, NH, et NR₂;
X est l'un quelconque de O ou S;
Z est l'un quelconque de S, CH₂, CHR₁ ou CR₁R₃;
W et Y sont indépendamment O, S, CH₂ or H₂;
R₁ et R₃ sont indépendamment un alkyle en C₁-c₆ à chaîne linéaire ou ramifiée ou un alcényle en C₂-C₆ à chaîne linéaire ou ramifiée,
où ledit alkyle ou alcényle est substitué avec un ou plusieurs substituants indépendamment choisis dans le groupe constitué par (Ar₁)ₙ, un alkyle en C₁-C₆ à chaîne linéaire ou ramifiée ou alcényle en C₂-C₆ à chaîne linéaire ou ramifiée substitué avec (Ar₁)ₙ, un, cycloalkyle en C₃-C₈, un alkyle en C₁-C₆ à chaîne linéaire ou ramifiée ou alcényle en C₂-C₆ à chaîne linéaire ou ramifiée substitué avec un cycloalkyle en C₃-C₈, et Ar₂;
n est 1 ou 2;
R₂ est l'un quelconque de un alkyle en C₁-C₉ à chaîne linéaire ou ramifiée, un alcényle en C₂-C₉ à chaîne linéaire ou ramifiée, un cycloalkyle en C₃-C₈, un cycloalcényle en C₅-C₇ ou Ar₁,
où ledit alkyle, alcényle, cycloalkyle ou cycloalcényle est soit non substitué, soit substitué avec un ou plusieurs substituants indépendamment choisis dans le groupe constitué par un alkyle en C₁-C₄ à chaîne linéaire ou ramifiée, un alcényle en C₂-C₄ à chaîne linéaire ou ramifiée et un hydroxy; et
Ar₁ et Ar₂ sont indépendamment un noyau carbo- ou hétérocyclique, mono-, bi- ou tricyclique, alicyclique ou aromatique,
où ledit noyau est soit non substitué, soit substitué avec un ou plusieurs substituants indépendamment choisis dans le groupe constitué par un halogène, un hydroxy, un nitro, un trifluorométhyle, un alkyle en C₁-C₆ à chaîne linéaire ou ramifiée, un alcényle en C₂-C₆ à chaîne linéaire ou ramifiée, un alcoxy en C₁-C₄, un alcényloxy en C₂-C₄, un phénoxy, un benzyloxy, et un amino,
où la taille des cycles individuels est de 5 - 8 membres,
et où le noyau hétérocyclique a 1 à 6 hétéroatomes indépendamment choisis dans le groupe constitué par O, N, et S.

3. Utilisation de la revendication 2, dans laquelle le noyau carbo- ou hétérocyclique, mono- ou bicyclique est choisi dans le groupe constitué par un naphtyle, un indolyle, un furyle, un thiazolyle, un thiényle, un pyridyle, un quinolinyle, un isoquinolinyle, un fluorényle, et un phényle.

4. Utilisation de la revendication 1, dans laquelle le composé est de la formule II 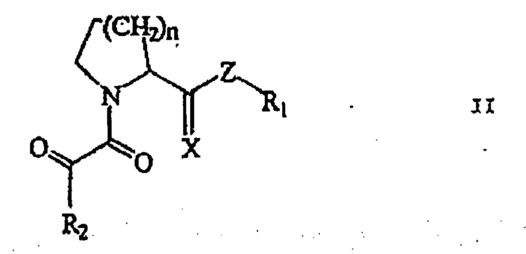 ou un sel, ester, ou produit de solvatation pharmaceutiquement acceptable de celle-ci, dans laquelle
n est 1 ou 2;
X est O ou S;
Z est choisi dans le groupe constitué par S, CH₂, CHR₁, et CR₁R₃;
R₁ et R₃ sont indépendamment choisis dans le groupe constitué par un alkyle en C₁-C₅ à chaîne linéaire ou ramifiée, un alcényle en C₂-C₅ à chaîne linéaire ou ramifiée, et Ar₁,
où ledit R₁ est non substitué ou substitué avec un ou plusieurs substituants indépendamment choisis dans le groupe constitué par un halogène, un nitro, un alkyle en C₁-C₆ à chaîne linéaire ou ramifiée, un alcényle en C₂-C₆ à chaîne linéaire ou ramifiée, un hydroxy, un alcoxy en C₁-C₄ un alcényloxy en C₂-C₄, un phénoxy, un benzyloxy, un amino, et Ar₁;
R₂ est choisi dans le groupe constitué par un alkyle en C₁-C₉ à chaîne linéaire ou ramifiée, un alcényle en C₂-C₉ à chaîne linéaire ou ramifiée, un cycloalkyle en C₃-C₈, un cycloalcényle en C₅-C₇ et Ar₁; et
Ar₁ est un phényle, un benzyle, un pyridyle, un fluorényle, un thioindolyle ou un naphtyle,
où ledit Ar₁ est non substitué ou substitué avec un ou plusieurs substituants indépendamment choisis dans le groupe constitué par un halogène, un trifluorométhyle, un hydroxy, un nitro, un alkyle en C₁-C₆ à chaîne linéaire ou ramifiée, un alcényle en C₂-C₆ à chaîne linéaire ou ramifiée, un alcoxy en C₁-C₄, un alcényloxy en C₂-C₄, un phénoxy, un benzyloxy, et un amino;

5. Utilisation de la revendication 4, dans laquelle :
n est 1; et
X est O.

6. Utilisation de la revendication 5, dans laquelle Z est CH₂.

7. Utilisation de la revendication 6, dans laquelle le composé est choisi dans le groupe constitué par :
la (2S)-2-({1-oxo-5-phényl}-pentyl-1-(3,3-diméthyl-1,2-dioxopentyl)pyrrolidine;
la 3,3-diméthyl-1-[(2S)-2-(5-(3-pyridyl)pentanoyl)-1-pyrrolidine]-1,2-pentanedione;
la (2S)-2-({1-oxo-4-phenyl}-butyl-1-(3,3-diméthyl-1,2-dioxobutyl)pyrrolidine;
la (2S)-2-({1-oxo-4-phényl}-butyl-1-(2-cyclohexyl-1,2-dioxoéthyl)pyrrolidine;
la 2-({1-oxo-4-phényl}-butyl-1-(3,3-diméthyl-1,2-dioxobutyl)pyrrolidine; et
la 1-((2S)-2-[5,5-di(4-fluorophényl)pentanoyl]-2-pyrrolidine}-3,3-diméthyl-1,2-pentanedione;
ou un sel, ester ou produit de solvatation pharmaceutiquement acceptable de ceux-ci.

8. Utilisation de la revendication 5, dans laquelle Z est S.

9. Utilisation de la revendication 8, dans laquelle le composé est choisi dans le groupe constitué par :
le (2S)-1-(3,3-diméthyl-1,2-dioxapentyl)-2-pyrrolidinecarbothioate de 2-phényl-1-éthyle ;
le (28)-1-(3,3-diméthyl-1,2-dioxopentyl)-2-pyrrolidinecarbothioate de (3-thioindolyl)mëthyle;
le (2S)-1-(2-cyclohexyl-1,2-dioxoéthyl)-2-pyrrolidinecarbothioate de 2-phényl-1-éthyle;
le (2S)-1-(1-cyclopentyl-1,2-dioxoéthyl)-2-pyrrolidinecarbothioate de 2-phényl-1-éthyle;
le (2S)-1-(3,3-dimëthyl-1,2-dioxopen,tyl)-2-pyrrolidinecarbothioate de..3-phényl-1-prQpyle;
le (2S)-1-(3,3-diméthyl-1,2-dioxoéthyl)-2-pyrrolidine-carbothioate de 3-(3-pyridyl)-1-propyle;
le (2S)-1- (2-cyclohexyl-1 2-dioxoëthyl) -2-pyrrolidinecarbothioate de 3-phényl-1-propyle;
le (2S)-1-(2-cycsohexyl-1,2-dioxoéthyl)-2-pyxrolidine-carbothioate de 4-phényl-1-butyle;
le (2S)-1-(3,3-diméthyl-1,2-dioxopentyl)-2-pyrrolidinecarbothioate de 4-phényl-1-butyle;
le (2S)-1-(2-cyclohexyl-1,2-dioxoéthyl)-2-pyrrolidinecarbothioate de 3-(3-pyridyl)-1-propyle;
le (2S)-1-(3,3-diméthyl-1,2-dioxopentyl)-2-pyrrolidinecarbothioate de 3,3-diphényl-1-propyle;
le (2S)-1-(2-cyclohexyl-1,2-dioxoéthyl)-2-pyrrolidinecarbothioate de 3,3-diphényl-1-propyle;
le (2S)-1-(3,3-diméthyl-1,2-dioxopentyl)-2-pyrrolidinecarbothioate de 3-(para-méthoxyphényl)-1-propyle;
le (2S)-1-(3,3-diméthyl-1,2-dioxopentyl)-2-pyrrolidinecarbothioate de 3,3-di(para-fluoro)phényl-1-propyle;
le 1-(3,3-diméthyl-2-oxopentanoyl)-2-pyrrolidinecarbothioate de 4,4 -di(para-fluorophényl)butyle;
le (2S)-1-(3,3-diméthyl-2-oxopentanoyl)-2-pyrrolidinecarbothioate de 3-(1-naphtyl)propyle;
le (2S)-1-(3,3-diméthyl-2-oxopentanoyl)tétrahydro-1H-2pyrrolidinecarbothioate de 2,2-diphényléthyle;
le (2S)-1-(3,3-diméthyl-2-oxopentanoyl)-2-pyrrolidinecarbothioate de 3-[4-(trifluorométhyl)phényl)propyle;
le (2S)-1-(3,3-diméthyl-2-oxopentanoyl)-2-pyrrolidinecarbothioate de 3-(2-naphtyl)propyle;
le (2S)-1-(3,3-diméthyl-2-oxopentanoyl)-2-pyrrolidinecarbothioate de 3-(3-chlorophényl)propyle;
le (2S)-1-(3,3-diméthyl-2-oxopentanoyl)-2-pyrrolidinecarbothioate de 3-(3-(trifluarométhyl) phényl]propyle;
le (2S)-1-(3,3-diméthyl-2-oxopentanoyl)-2-pyrrolidinecarbothioate de 3-(1-biphényl)propyle;
le (2S)-1-(3,3-diméthyl-2-oxopentanoyl)-2-pyrrolidinecarbothioate de 3-(2-fluorophényl)propyle;
le (2S)-1-(3,3-diméthyl-2-oxopentanoyl)-2-pyrrolidinecarbothioate de 3-(3-fluorophényl)propyle;
le (2S)-1-(3,3-diméthyl-2-oxopentanoyl)-2-pyrrolidinecarbothioate de 3-(2-chlorophényl) propyle; et
le (2S)-1-(3,3-diméthyl-2-oxopentanoyl)-2-pyrrolidinecarbothioate de 3-(3,4-diméthoxyphényl)propyle;
ou un sel, ester, ou produit de solvatation pharmaceutiquement acceptable de ceux-ci.

10. Utilisation de la revendication 9; dans laquelle le composé est le (2S)-1-(3,3-diméthyl-1,2-dioxopentyl)-2-pyrrolidinecarbothioate de 3,3-diphényl-1-propyle ou un sel, ester, ou produit de solvatation pharmaceutiquement acceptable de celui-ci.

11. Utilisation de la revendication 9, dans laquelle le composé est le (2S)-1-(3,3-diméthyl-2-oxopentanoyl)-2-pyrrolidinecarbothioate de 3-(1-naphtyl)propyle ou un sel, ester, ou produit de solvatation pharmaceutiquement acceptable de celui-ci.

12. Utilisation de la revendication 9, dans laquelle le composé est le (2S)-1-(3,3-diméthyl-2-oxopentanoyl)-tétrahydro-1H-2-pyrrolidinecarbothioate de 2,2-diphényléthyle ou un sel, ester, ou produit de solvatation pharmaceutiquement acceptable de celui-ci.

13. Utilisation de la revendication 9, dans laquelle le composé est le (2S)-1-(3,3-diméthyl-2-oxapentanayl)-2-pyrxolidinecarbothioate de 3-(4-(trifluorométhyl)phényl)-propyle ou un sel, ester, ou produit de solvatation pharmaceutiquement acceptable de celui-ci.

14. Utilisation de la revendication 9, dans laquelle le composé est le (2S)-1-(3,3-diméthyl-2-oxopentanoyl)-2-pyrrolidinecarbothioate de 3-(2-naphtyl)propyle ou un sel, ester, ou produit de solvatation pharmaceutiquement acceptable de celui-ci.

15. Utilisation de la revendication 4, dans laquelle :
n est 1; et
X est S.

16. Utilisation de la revendication 15, dans laquelle Z est CH₂.

17. Utilisation de la revendication 15, dans laquelle Z est S.

18. Utilisation de la revendication 4, dans laquelle :
n est 2; et
X est o.

19. Utilisation de la revendication 18, dans laquelle Z est CH₂.

20. Utilisation de la revendication 19, dans laquelle le composé est choisi dans le groupe constitué par :
la 2-({1-oxo-6-phényl}-hexyl-1-(2-cyclohexyl-1,2-dioxoxyéthyl)pipéridine; et
la 3,3-diméthyl-1-(2-(4-phénylpentanoyl)pipéridino]-1,2-pentanedione;
ou un sel, ester, ou produit de solvatation pharmaceutiquement acceptable de celles-ci

21. Utilisation de la revendication 18, dans laquelle Z est S.

22. Utilisation de la revendication 21, dans laquelle le composé est choisi dans le groupe constitué par :
le 1-(3,3-diméthyl-1,2-dioxopentyl)-2-pipéridine-carbothioatè de 2-phényl-1-éthyle;
le 1-(2-phén.yl-1,2-dioxoéthyl)-2-pipéridinecarbothioate de 2-phényl-1-éthyle;
le 1-(3,3-diméthyl-1,2-dioxabutyl)-2-pipéridinecarbothioate de 3-phényl-1-propyle;
le 1-(1,2-dioxo-3,3-diméthylbutyl)-2-pipéridinecarbothioate de 4-phényl-1-butyle;
le 1-(3,3-diméthyl-1,2-dioxopentyl)-2-pipéridinecarbothioate de 1,5-diphényl-3-pentyle;
le 1-(3-phényl-1,2-dioxoéthyl)-2-pipéridinecarbothioate de 1,5-diphényl-3-mercaptopentyle;
le 1-(1,2-dioxo-3,3-diméthylpentyl)pipéridine-2carbothioate de 3-(para-méthoxyphényl)-1-propyle;
le 1-(2-phényl-1,2-dioxoéthyl)pipéridine-2-carbothioate de 3-(para-méthoxyphényl)-1-propyle;
le 1-(3,3-diméthyl-1,2-dioxopentyl)pipéridine-2-carbothioate de 3-(1-naphtyl)-1-propyle;
le (2S)-1-(3,3-diméthyl-2-oxopentanoyl)-2-pipéridinecarbothioate de 2,2-diphényléthyle;
le 1-(3,3-diméthyl-2-oxopentanoyl)-2-pipéridinecarbothioate de 3,3-diphénylpropyle;
le (2R,S)-1-(3,3-diméthyl-2-oxopentanoyl)-2-pipéridinecarbothioate de 3-(2-naphtyl)propyle;
le 1-(3,3-dimëthyl-2-oxopentanoyl)-2-pxpéridinecarbothioate de 4-phénylbutyle;
le 1-(3,3-diméthyl-2-oxopentanoyl)-2-pipéridinecarbothioate de 3-phénylpropyle;
le 1-(3,3-diméthyl-2-oxopentanoyl)-2-pipéridinecarbothioate de 3-(2-chlorophényl)propyle;
le 1-(3,3-diméthyl-2-oxopentanoyl)-2-pipéridinecarbothioate de 3-(2-fluorophényl)propyle; et
le 1-(3,3-diméthyl-2-oxopentanoyl)-2-pipéridinecarbothioate de 3-(3-fluoraphényl)propyle;
ou un sel, ester, ou produit de solvatation pharmaceutiquement acceptable de ceux-ci

23. Utilisation de la revendication 22, dans laquelle le composé est le 1-(3,3-diméthyl-2-oxopentanoyl)-2-pipéridinecarbothioate de 3,3-diphénylpropyle ou un sel, ester, ou produit de solvatation pharmaceutiquement acceptable de celui-ci.

24. Utilisation de la revendication 4, dans laquelle :
n est 2; et
x est S.

25. Utilisation de la revendication 24, dans laquelle Z est CH₂.

26. Utilisation de la revendication 24, dans laquelle Z est S.

27. Utilisation de la revendication 24, dans laquelle Z est CHR₁.

28. Utilisation de la revendication 27, dans laquelle le composé est la 2-({1-oxo-[2-{2'-phényl}éthyl]-4-phényl}-butyl-1-(3,3-diméthyl-1,2-dioxobutyl)pipéridine, ou un sel, ester, ou produit de solvatation pharmaceutiquement acceptable de celle-ci

29. Utilisation de la revendication 1, dans laquelle le composé est de la formule III 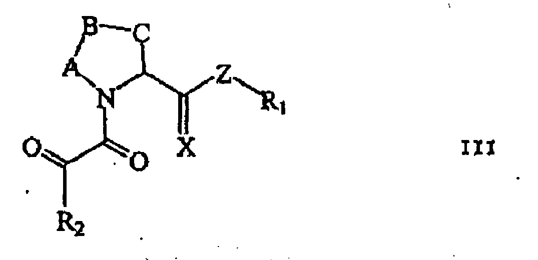 ou un sel, ester, ou produit de solvatation pharmaceutiquement acceptable de celle-ci, dans laquelle .
A, B, et C sont indépendamment CH₂, O, S, SO, SO₂, NH ou NR₂ ;
X est O ou S;
Z est S, CH₂, CHR₁ ou CR₁R₃ ;
R₁ et R₃ sont indépendamment un alkyle en C₁-C₆ à chaîne linéaire ou ramifiée ou un alcényle en C₂-C₆ à chaîne linéaire ou ramifiée,
où ledit alkyle ou alcényle est substitué avec un ou plusieurs substituante indépendamment choisis dans le groupe constitué par (Arᵢ)ₙ, un alkyle en C₁-C₆ à chaîne linéaire ou ramifiée ou alcényle en C₂-C₆ à chaîne linéaire ou ramifiée substitué avec (Ar₁)ₙ, un cycloalkyle en C₃-C₈, un alkyle en C₁-C₆ à chaîne linéaire ou ramifiée ou alcényle en C₂-C₆ à chaîne linéaire ou ramifiée substitué avec un cycloalkyle en C₃-C₈, et Ar₂;
R₂ est l'un quelconque de un alkyle en C₁-C₉ à chaîne linéaire ou ramifiée, un alcényle en C₂-C₉ à chaîne linéaire ou ramifiée, un cycloalkyle en C₃-C₈, un cycloalcényle en C₅-C₇ ou Ar₁,
où ledit alkyle, alcényle, cycloalkyle ou cycloalcényle est soit non substitué, soit substitué avec un ou plusieurs substituants indépendamment choisis dans le groupe constitué par un alkyle en C₁-C₄ à chaîne linéaire ou ramifiée, un alcényle en C₂-C₄ à chaîne linéaire ou ramifiée et un hydroxy; et
Ar₁ et Ar₂ sont indépendamment un noyau carbo- ou hétérocyclique, mono-, bi- ou tricyclique, alicyclique ou aromatique,
où ledit noyau est soit non substitué, soit substitué avec un ou plusieurs substituants indépendamment choisis dans le groupe constitué par un halogène, un hydroxy, un nitro, un trifluorométhyle, un alkyle en C₁-C₆ à chaîne linéaire ou ramifiée, un alcényle en C₂-C₆ à chaîne linéaire ou ramifiée, un alcoxy en C₁-C₄, un alcényloxy en C₂-C₄, un phénoxy, un benzyloxy, et un amino,
où la taille des cycles individuels est de 5 - 8 membres,
et où le noyau hétérocyclique a 1 à 6 hétéroatomes indépendamment choisis dans le groupe constitué par O, N, et S.

30. Utilisation de la revendication 29, dans laquelle .
A est CH₂;
B est CH₂ ou S;
C est CH₂ ou NH;
X est O ou S;
Z est S ou CH₂;
R₁ est un 2-phenéthyle ou un 3-phénylpropyle; et
R₂ est un 1,1-diméthylpropyle.

31. Utilisation de la revendication 1, dans laquelle le composé est de la formule IV 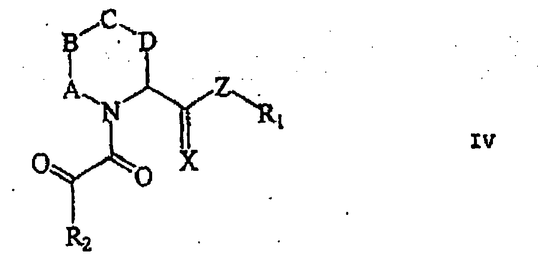 ou un sel, ester, ou produit de solvatation de celle-ci, dans laquelle
A, B, C et D sont indépendamment CH₂, O, S, SO, SO₂, NH, ou NR₂;
X est O ou S;
Z est S, CH₂, CHR₁, ou CR₁R₃ ;
R₁ et R₃ sont indépendamment un alkyle en C₁-C₆ à chaîne linéaire ou ramifiée ou un alcényle en C₂-C₆ à chaîne linéaire ou ramifiée,
où ledit alkyle ou alcényle est substitué avec un ou plusieurs substituants indépendamment choisis dans le groupe constitué par (Ar₁)ₙ, un alkyle en C₁-C₆ à chaîne linéaire ou ramifiée ou alcényle en C₂-C₆ à chaîne linéaire ou ramifiée substitué avec (Ar₁)ₙ, un cycloalkyle en C₃-C₈, un alkyle en C₁-C₅ à chaîne linéaire ou ramifiée ou alcényle en C₂-C₆ à chaîne linéaire ou ramifiée substitué avec un cycloalkyle en C₃-C₈, et Ar₂;
n est 1 ou 2;
R₂ est l'un quelconque de un alkyle en C₁-C₉ à chaîne linéaire ou ramifiée, un alcényle en C₂-C₉ à chaîne linéaire ou ramifiée, un cycloalkyle en C₃-C₈, un cycloalcényle en C₅-C₇ ou Ar₁,
où ledit alkyle, alcényle, cycloalkyle ou cycloalcényle est soit non substitué, soit substitué avec un ou plusieurs substituants indépendamment choisis dans le groupe constitué par un alkyle en C₁-C₄ à chaîne linéaire ou ramifiée, un alcényle en C₂-C₄ à chaîne linéaire ou ramifiée et un hydroxy; et
Ar₁ et Ar₂ sont indépendamment un noyau carbo- ou hétérocyclique, mono-, bi- ou tricyclique, alicyclique ou aromatique,
où ledit noyau est soit non substitué, soit substitué avec un ou plusieurs substituants indépendamment choisis dans le groupe constitué par un halogène, un hydroxy, un nitro, un trifluorométhyle, un alkyle en C₁-C₆ à chaîne linéaire ou ramifiée, un alcényle en C₂-C₆ à chaîne linéaire ou ramifiée, un alcoxy en C₁-C₄, un alcényloxy en C₂-C₄, un phénoxy, un benzyloxy, et un amino,
où la taille des cycles individuels est de 5 - 8 membres,
et où le noyau hétérocyclique a 1 à 6 hétéroatomes indépendamment choisis dans le groupe constitué par O, N, et S.

32. Utilisation de la revendication 31, dans laquelle :
A est CH₂;
B est CH₂;
C est S ou O;
D est CH₂;
X est 0;
Z est CH₂ ou S;
R₁ est un 3-phénylpropyle ou un 2-phénéthyle; et
R₂ est un 1,1-diméthylpropyle.

33. Utilisation de l'une quelconque des revendications 1 à 32, dans laquelle le composé est non-immunosuppresseur.

34. Utilisation de l'une quelconque des revendications 1 à 32, dans laquelle le composé a une affinité pour une immunophiline de type FKBP.

35. Utilisation de la revendication 34, dans laquelle l'immunophiline de type FKBP est la FKBP-12.

36. Utilisation de l'une quelconque des revendications 1 à 32, dans laquelle le composé est immunosuppresseur.
